# EUROPEAN PATENT APPLICATION

(11) **EP 4 043 011 A1**
(43) Date of publication of application: **17.08.2022**
(21) Application number: 21190327.3
(22) Date of filing: 15.05.2015
(51) Int. Cl.: A61K 31/145, C12Q 1/68, G01N 33/53

(54) **METHODS FOR ASSESSING RESPONSIVENESS TO ASTHMA TREATMENT BASED ON VNN-1 EXPRESSION AND PROMOTER METHYLATION**

(30) Priority: 16.05.2014 US 201461994477 P
(62) Divisional of application: 15792604.9
(71) Applicant: Children's Hospital Medical Center, Cincinnati, Ohio 45229-3029 (US)
(72) Inventor: HERSHEY, Gurjit, Khurana, Cincinnati, OH 45249 (US); MYERS, Jocelyn, Biagini, Cincinnati, OH 45245 (US); JI, Hong, Blue Ash, OH 45241 (US); MARTIN, Lisa, J., West Chester, OH 45069 (US)
(74) Representative: Potter Clarkson

(57) **Abstract**

Provided herein are methods and kits related to use of vanin-1 (VNN1) expression for assessing responsiveness to steroid treatment in subjects with asthma and for treating subjects with asthma.

SEQUENCE LISTING

<110>  Children's Hospital Medical Center

<120>  METHODS FOR ASSESSING RESPONSIVENESS TO ASTHMA TREATMENT BASED ON
       VNN-1 EXPRESSION AND PROMOTER METHYLATION

<130>  B2380EPPC01

<150>  US 61/994,477
<151>  2014-05-16

<160>  20

<170>  PatentIn version 3.5

<210>  1
<211>  3844
<212>  DNA
<213>  Homo sapiens

<400>  1
agcactcatt ggacttcagc atgactactc agttgccagc ttacgtggca attttgcttt      60

tctatgtctc aagagccagc tgccaggaca ctttcactgc agctgtttat gagcatgcag     120

cgatattgcc caatgccacc ctaacaccag tgtctcgtga ggaggctttg gcattaatga     180

atcggaatct ggacattttg gaaggagcga tcacatcagc agcagatcag ggtgcgcata     240

ttattgtgac tccagaagat gctatttatg gctggaactt caacagggac tctctctacc     300

catatttgga ggacatccca gaccctgaag taaactggat cccctgtaat aatcgtaaca     360

gatttggcca gaccccagta caagaaagac tcagctgcct ggccaagaac aactctatct     420

atgttgtggc aaatattggg gacaagaagc catgcgatac cagtgatcct cagtgtcccc     480

ctgatggccg ttaccaatac aacactgatg tggtatttga ttctcaagga aaactggtgg     540

cacgctacca taagcaaaac cttttcatgg gtgaaaatca attcaatgta cccaaggagc     600

ctgagattgt gactttcaat accacctttg gaagttttgg cattttcaca tgctttgata     660

tactcttcca tgatcctgct gttaccttgg tgaaagattt ccacgtggac accatagtat     720

tcccaacagc ttggatgaat gttttgccac atttgtcagc tgttgaattc cactcagctt     780

gggctatggg catgagggtc aatttccttg catccaacat acattacccc tcaaagaaaa     840

tgacaggaag tggcatctat gcacccaatt cttcaagagc atttcattat gatatgaaga     900

cagaagaggg aaaactcctc ctctcgcaac tggattccca cccatcccat tctgcagtgg     960

tgaactggac ttcctatgcc agcagtatag aagcgctctc atcaggaaac aaggaattta    1020

aaggcactgt ctttttcgat gaattcactt ttgtgaagct cacaggagtt gcaggaaatt    1080

atacagtttg tcagaaagat ctctgctgtc atttaagcta caaaatgtct gagaacatac    1140

caaatgaagt gtacgctcta ggggcatttg acggactgca cactgtggaa gggcgctatt    1200

atctacagat ttgtaccctg ttgaaatgta aaacgactaa tttaaacact tgcggtgact    1260

cagctgaaac agcttctacc aggtttgaaa tgttctccct cagtggcact ttcggaaccc 1320

agtatgtctt tcctgaggtg ttgctgagtg aaaatcagct tgcacctgga gaatttcagg 1380

tgtcaactga cggacgcttg tttagtctga agccaacatc cggacctgtc ttaacagtaa 1440

ctctgtttgg gaggttgtat gagaaggact gggcatcaaa tgcttcatca ggcctcacag 1500

cacaagcaag aataataatg ctaatagtta tagcacctat tgtatgctca ttaagttggt 1560

agaatattga ctttttctct tttttatttg ggataattta aaaaatgatg gatgagaaaa 1620

gaaagattgg tccgggttaa tattatcctc tagtataagt gaattactag tttctcttta 1680

tttagacaaa cacacacaca ccagataata taaacttaat aaattatctg ttaatgtaga 1740

ttttatttaa aaaactatat ttgaacattg gtctttcttg gacgtgagct aattatatca 1800

aataagtatc acaaatcttt tacgcagaag aaataaaaac tacgggtaga aaacataaga 1860

actatcataa aatttactta caaggaggct gctcttgtta ccacttttat tatattacgt 1920

atcacttatt cagctctgct gaaaatttcc aatgactttg tttgtttgct ctttttgttt 1980

tttacctaaa caatacattt tgattctctt gtgggttgat aatgtctccc caaaatttac 2040

atgttgaagc acctcagaat gtgactgtat ttggagacag ggtctttaaa gaggtaaaat 2100

aaggtcatta ggatagaccc taattcaata tgactgatga tcataaaaga agaggcgagt 2160

agggcacaac aggcacaaag ggagaccata aggagacaca gaggaaggac aactctttac 2220

aagctaagaa gagagggcct cagaagaaac caaccctgcc aacaccttga tcttggactt 2280

ccagcctcca aaactatgag aaataaattt ctattgttta agtcacccag tccatggtac 2340

tttgttaggc agccctggca aatgaatcaa agacccattc ctgttcctct ccccaccact 2400

actgttttct actgtaatct gaagcttcaa caaaaggctt acctggtaag aatattcagc 2460

tggtctgggt cctcaagact ccaatagaca ctcttagaga aggattgctg atggattgat 2520

agtgaaacca ttagatcatt gaattcctct ggaattagaa aaccagagag tcccatttta 2580

agaaattaga tatttaatat agcattgtgt gttctatttt agtaacagca gaatctcttg 2640

acattacaca actcagtgaa acaacatcat ttaagccaaa atatctccca actgactgat 2700

agactctgag cactaatatc atagtgctgt gatgatggac aattacatag taccgataac 2760

agccatgcac tgtgcaaagc atgcccttct gcacaggaga gcaaggcact tgcagtagtg 2820

atctatgcca gcaaaacatc attttgagac aaacattttt gtggcagatg tttttcctaa 2880

aaagtactat atcatccaag aaatatttga gtaaaatccc ttgttctttt gggtgacatt 2940

aactgacatt tgcttttttt caagacctaa tagaaaataa gaaagcccat aatgtattta 3000

gaaacaggaa tcctcagagc aattctctgt attctcatat aatttcaatg taaaacagaa 3060

aacatattga tgtgttggtg ataggcttga attattaaaa acttcaaaaa catcctaagt 3120

gtttcttttt tgctcaacgt tgtcaactat agtaggtctc ccttgtggtg taatgaattg 3180

cccccaaact attatcttaa aacaacaaac atttattatc ttatagcatt tctgagggtc 3240

aggatctggg actggcttag tggagttgtt ctggatcagg gcctttggaa agttgtagtt 3300

aacttgtccc cagggctgcc atcatctcaa ggctcgggtg gggctggaga aaatctgctt 3360

ctcagctcac tcacggcggt tgccaggcct ccattcttta ggatgctaga aaaactttca 3420

taaaatgtca tctggcttct cctagagcaa tgatactgag agagaaagca catgagagaa 3480

agagcgaggg aacttggatg taagccacag tctttgaaaa cctaatcaca gaagtgacat 3540

ctcttcttcc acatgatgtt ggtcacatgg accaacaatg gcacaacgtg gacagaatca 3600

aacagagttg agaatatcag gaggtggggc ttcatggggg ccattttgga tgctatcata 3660

gtgaatatat gtatttatat ttatatctgt atatattgca atgtaattta aaaaatagga 3720

ttgttttcct tttctttttg ctatatgtga tatgtatttc aaaatacact cccaatagtt 3780

acgtctgaaa agcactacac taaaaaactt tctatacatt gaataattaa attaaataat 3840

ctaa 3844

<210> 2
<211> 513
<212> PRT
<213> Homo sapiens

<400> 2

Met Thr Thr Gln Leu Pro Ala Tyr Val Ala Ile Leu Leu Phe Tyr Val
1               5                   10                  15

Ser Arg Ala Ser Cys Gln Asp Thr Phe Thr Ala Ala Val Tyr Glu His
            20                  25                  30

Ala Ala Ile Leu Pro Asn Ala Thr Leu Thr Pro Val Ser Arg Glu Glu
        35                  40                  45

Ala Leu Ala Leu Met Asn Arg Asn Leu Asp Ile Leu Glu Gly Ala Ile
    50                  55                  60

Thr Ser Ala Ala Asp Gln Gly Ala His Ile Ile Val Thr Pro Glu Asp
65                  70                  75                  80

Ala Ile Tyr Gly Trp Asn Phe Asn Arg Asp Ser Leu Tyr Pro Tyr Leu
                85                  90                  95

Glu Asp Ile Pro Asp Pro Glu Val Asn Trp Ile Pro Cys Asn Asn Arg
            100                 105                 110

Asn Arg Phe Gly Gln Thr Pro Val Gln Glu Arg Leu Ser Cys Leu Ala

115                 120                 125

Lys Asn Asn Ser Ile Tyr Val Val Ala Asn Ile Gly Asp Lys Lys Pro
    130                 135                 140

Cys Asp Thr Ser Asp Pro Gln Cys Pro Pro Asp Gly Arg Tyr Gln Tyr
145                 150                 155                 160

Asn Thr Asp Val Val Phe Asp Ser Gln Gly Lys Leu Val Ala Arg Tyr
                165                 170                 175

His Lys Gln Asn Leu Phe Met Gly Glu Asn Gln Phe Asn Val Pro Lys
            180                 185                 190

Glu Pro Glu Ile Val Thr Phe Asn Thr Thr Phe Gly Ser Phe Gly Ile
        195                 200                 205

Phe Thr Cys Phe Asp Ile Leu Phe His Asp Pro Ala Val Thr Leu Val
    210                 215                 220

Lys Asp Phe His Val Asp Thr Ile Val Phe Pro Thr Ala Trp Met Asn
225                 230                 235                 240

Val Leu Pro His Leu Ser Ala Val Glu Phe His Ser Ala Trp Ala Met
                245                 250                 255

Gly Met Arg Val Asn Phe Leu Ala Ser Asn Ile His Tyr Pro Ser Lys
            260                 265                 270

Lys Met Thr Gly Ser Gly Ile Tyr Ala Pro Asn Ser Ser Arg Ala Phe
        275                 280                 285

His Tyr Asp Met Lys Thr Glu Glu Gly Lys Leu Leu Leu Ser Gln Leu
    290                 295                 300

Asp Ser His Pro Ser His Ser Ala Val Val Asn Trp Thr Ser Tyr Ala
305                 310                 315                 320

Ser Ser Ile Glu Ala Leu Ser Ser Gly Asn Lys Glu Phe Lys Gly Thr
                325                 330                 335

Val Phe Phe Asp Glu Phe Thr Phe Val Lys Leu Thr Gly Val Ala Gly
            340                 345                 350

Asn Tyr Thr Val Cys Gln Lys Asp Leu Cys Cys His Leu Ser Tyr Lys
        355                 360                 365

Met Ser Glu Asn Ile Pro Asn Glu Val Tyr Ala Leu Gly Ala Phe Asp
    370                 375                 380

Gly Leu His Thr Val Glu Gly Arg Tyr Tyr Leu Gln Ile Cys Thr Leu
385                 390                 395                 400

Leu Lys Cys Lys Thr Thr Asn Leu Asn Thr Cys Gly Asp Ser Ala Glu
                405                 410                 415

Thr Ala Ser Thr Arg Phe Glu Met Phe Ser Leu Ser Gly Thr Phe Gly
            420                 425                 430

Thr Gln Tyr Val Phe Pro Glu Val Leu Leu Ser Glu Asn Gln Leu Ala
        435                 440                 445

Pro Gly Glu Phe Gln Val Ser Thr Asp Gly Arg Leu Phe Ser Leu Lys
    450                 455                 460

Pro Thr Ser Gly Pro Val Leu Thr Val Thr Leu Phe Gly Arg Leu Tyr
465                 470                 475                 480

Glu Lys Asp Trp Ala Ser Asn Ala Ser Ser Gly Leu Thr Ala Gln Ala
                485                 490                 495

Arg Ile Ile Met Leu Ile Val Ile Ala Pro Ile Val Cys Ser Leu Ser
            500                 505                 510

Trp

<210>  3
<211>  2000
<212>  DNA
<213>  Homo sapiens

<400>  3
agcagttaga gaagctggtg tttaccggaa actcaggaga accagccaac cacaacccct      60

gggttacctt ggcaattgca gaataaatgc attatagtta ctaaagtaaa aaattagata     120

tgcctgtttg cagattgaac tataaaaata ccattcaaag acaaatagat ctaaaaataa     180

aatggaaaaa cataaacact aattctgtaa atattatact taatgcacaa ctgaaacaaa     240

atttgccagc ttactcaata tcaaaatcta tgaacagttt ttctatttta tataatttcc     300

ctctcctctc tctggatctc gctccccagc tcattttttc ttttttttgc tctgattctt     360

tatacacctc tgttgcctct gtgataagca gcttcaaaga tggttcctaa tgctttattg     420

gatagaatac aacaaaagcg atgaggtgtt gcttccccaa ttacattacg aagcatccgt     480

ggcttccatc tccagtgggt tcacttgctg tctggctcta agggaatcca gataccataa     540

tgcgggctgc cctatggtga ggtttgcatc actaggaact catgtctctg ggcaacaacc 600

aatgaggtct tgatccctgc cgtcagccac atgagggagc ttggagctcg gaagtgaatc 660

ctcctggagt caagccttga tatagctagc cctggcagct gcttgactgc agccttgtga 720

aagagacctt gggccagagg caccagctaa actgcccctg gattcctgac ccagagaaag 780

tgggagatga tgtatttttg ctttttgaag ctgctgaatt tggggataat ttgttatata 840

gcaatagaaa atgagtaact cttttgtatt cctctttgtc ctggcttccc cattttgagg 900

aaaataaagt aaatcaaagt gtagagctga aatattcaca tgaaaataat aataaagttt 960

taaaattatt tgaatgtctt gtgttgacat tccaaaatat atgaattcca aaaatttata 1020

tgttgaagtc ctaactgtca gtatcttaga atgtaacttt tttggaaaag gggtcatttc 1080

agatctaatt agttaagatg aagttatact ggagtacagt gggcactaaa tcgaattggt 1140

cctatgattg agtctcagtc tttcagtgag cctgtacccc tgggtttatg accttcagtt 1200

ggcttttttc ttctgccctt atttggcata aaaacaaagc aggtggatca cctgaggtca 1260

gcaatttgag accagcctgc ccaacacggc gaaaccctat ctctactaaa aatacaaaaa 1320

attagcctgg cgtggtggcg ggcgcctgta atcccagcta cttgggaggc tgaggcagga 1380

gaatcacatg aacccgagag gcggaggttg cagtgagccg agatttcgcc actgcactct 1440

agcctgggtg acaagagtga aactccatct caaacaacaa caacaataaa caaacaacaa 1500

cgatgacaaa aaaagctaga gctgggattt tccctttccc tgtgttaaag attagagtgg 1560

tgtcctcaca aaaagggaaa acttggatac aggcacacac atggggagaa tagcatatga 1620

agagacacag ggagaaggca gccatctatg ggtcaaggag agaggcctgg aacacatctt 1680

tccttcaccg ccctcaggag gaaccaactc tgctgacacc ttcatctggg actcccaccc 1740

tccagaactg caaagcaata aattttttat tttttacacc acccagttta ttgtattttg 1800

ttaggcagcc ctagcgaact aatgtacata gagttcttga gttaatcttc acaaattact 1860

gcaataaggt agggtctttt gttatgtaac aatgctatga aatcatagcg ttttcttaat 1920

taacttccgt agtttaaggt actaagttct ggacaccacg tgtcttcttt ctataaatac 1980

caggacatgc tctgtttttc 2000

<210> 4
<211> 19
<212> DNA
<213> Artificial sequence

<220>
<223> Primer sequence

<400> 4
cctggttctg gaatcctca 19

<210>  5
<211>  22
<212>  DNA
<213>  Artificial sequence

<220>
<223>  Primer sequence

<400>  5
tcgaacattg gtccttttc tt                                                22


<210>  6
<211>  20
<212>  DNA
<213>  Artificial sequence

<220>
<223>  Primer sequence

<400>  6
ttacagccca gatagctctt                                                  20


<210>  7
<211>  17
<212>  DNA
<213>  Artificial sequence

<220>
<223>  Primer sequence

<400>  7
atggcttcca gcaactc                                                     17


<210>  8
<211>  20
<212>  DNA
<213>  Artificial sequence

<220>
<223>  Primer sequence

<400>  8
tctgcatccc tggtgtgtaa                                                  20


<210>  9
<211>  19
<212>  DNA
<213>  Artificial sequence

<220>
<223>  Primer sequence

<400>  9
aagttgatgg cttcaggag                                                   19


<210>  10
<211>  17

<212>  DNA
<213>  Artificial sequence

<220>
<223>  Primer sequence

<400>  10
ctcagtggca ctttcgg                                                    17


<210>  11
<211>  21
<212>  DNA
<213>  Artificial sequence

<220>
<223>  Primer sequence

<400>  11
caacctccca aacagagtta c                                               21


<210>  12
<211>  23
<212>  DNA
<213>  Artificial sequence

<220>
<223>  Primer sequence

<400>  12
ggggaaggtg aaggtcggag tca                                             23


<210>  13
<211>  22
<212>  DNA
<213>  Artificial sequence

<220>
<223>  Primer sequence

<400>  13
agccttgacg gtgccatgga at                                              22


<210>  14
<211>  25
<212>  DNA
<213>  Artificial sequence

<220>
<223>  Primer sequence

<400>  14
aggtgttgtt tttttaatta tatta                                           25


<210>  15
<211>  25
<212>  DNA
<213>  Artificial sequence

<220>
<223>  Primer sequence

<400>  15
cttaactcca aaaaaattca cttcc                                          25


<210>  16
<211>  28
<212>  DNA
<213>  Artificial sequence

<220>
<223>  Primer sequence

<400>  16
tttaaagatg gtttttaatg ttttattg                                       28


<210>  17
<211>  25
<212>  DNA
<213>  Artificial sequence

<220>
<223>  Primer sequence

<400>  17
cccaaaatct ctttcacaaa actac                                          25


<210>  18
<211>  25
<212>  DNA
<213>  Artificial sequence

<220>
<223>  Primer sequence

<400>  18
aggtgttgtt tttttaatta tatta                                          25


<210>  19
<211>  25
<212>  DNA
<213>  Artificial sequence

<220>
<223>  Primer sequence

<400>  19
gttgtttggt tttaagggaa tttag                                          25


<210>  20
<211>  25
<212>  DNA
<213>  Artificial sequence

<220>
<223>  Primer sequence

<400> 20
ttgggtaata attaatgagg ttttg 25

## Description

### BACKGROUND OF THE INVENTION

Asthma affects 25.7 million people in the US including 7.0 million children. Akinbami et al., NCHS data brief 2012:1-8. Although patients suffering from asthma share similar clinical symptoms, the disease is heterogeneous. Bel, The New England journal of medicine 2013;369:2362. This heterogeneity contributes to the difficulty in both studying and treating asthma. Nearly two-thirds of children who currently have asthma reported at least one attack in the previous 12 months (Fassl et al., Pediatrics 2012;130:482-91), highlighting the suboptimal management of asthma in this age group (Akinbami, Advance data 2006:1-24). The frequency of absent or incomplete efficacy in asthma treatment has been estimated to be 40-70%. Drazen et al., British medical bulletin 2000;56:1054-70. Up to 27% of children admitted for asthma exacerbation require longer than a three-day stay and this phenotype seems to be conserved within a given individual and is partially heritable. Akinbami, 2006; and Morray et al., Archives of pediatrics & adolescent medicine 1995;149:276-9. Thus, this may represent a distinct phenotype of asthma that is poorly responsive to standard treatment regimens for inpatient asthma.

Considerable advances have been made in recent years in identifying subphenotypes of asthma including treatment response phenotypes. One phenotype, associated with high interleukin-13 expression and increased circulating periostin, is associated with improved response to an interleukin-13 inhibitor and to anti-IL-4 receptor alpha therapy. Woodruff et al., American journal of respiratory and critical care medicine 2009;180:388-95; Corren et al., The New England journal of medicine 2011;365:1088-98; and Wenzel et al., The New England journal of medicine 2013;368:2455-66. Further, genetic variation in *GLCCI1* (glucocorticoid-induced transcript-1) was associated with response to inhaled glucocorticoids. Tantisira et al., The New England journal of medicine 2011;365:1173-83. Another study examining gene expression changes in response to inhaled corticosteroids in asthmatic adults using airway epithelial cells identified that high baseline expression of three genes (CLCA1, periostin and serpinB2) and one gene (FKBP51) were associated with good or poor clinical response to corticosteroids, respectively. Woodruff et al., PNAS, 2007;104:15858-63. In a recent study, expression of 6 genes in induced sputum discriminated between eosinophilic and neutrophilic asthma and predicted response to treatment with inhaled corticosteroids. Baines et al., J Allergy Clin Immunol., 2014;133:997-1007.

### SUMMARY OF THE INVENTION

Aspects of the disclosure relate to biomarkers for assessing responsiveness to asthma treatments in subjects with asthma and application of such biomarkers in determining treatment strategy for a subject in need of the treatment. The disclosure is based, in part, on the unexpected discovery that vanin 1 (VNN1) expression levels (including mRNA levels and/or methylation levels in the promoter region of a VNN1 gene in, *e.g.,* nasal epithelial cells) can stratify subjects with asthma as good responders or poor responders to steroid treatment. It was found that decreased levels of VNN1 mRNA and decreased CpG methylation of the VNN1 promoter (e.g., methylation of CpG4) correlated with a poor response to steroid treatment. This was verified in an animal model of asthma, where animals lacking the VNN1 gene responded poorly to steroid treatment, while animals with the wild-type VNN1 gene responded well to steroid treatment.

In one aspect, the present disclosure provides a method of assessing responsiveness to a steroid treatment of asthma in a subject, the method comprising: (a) measuring a level of Vanin 1 (VNN1) expression in a biological sample obtained from a subject having asthma, suspected of having asthma, or at risk for asthma; and (b) assessing the subject's responsiveness to the steroid treatment based on the level of VNN1 expression. A decreased level of VNN1 expression relative to a pre-determined value indicates that the subject is not responsive or not likely to respond to the steroid treatment. The same or an elevated level of VNN1 expression relative to the pre-determined value indicates that the subject is responsive or likely to respond to the steroid treatment.

In some embodiments, the level of VNN1 expression is represented by a level of VNN1 mRNA in the biological sample of the subject, which may be measured by an array-based assay or a PCR-based assay. In other embodiments, the level of VNN1 expression is represented by a level of CpG methylation in the promoter of a VNN1 gene. For example, the level of CpG methylation is the methylation level of CpG4 in the promoter of the VNN1 gene. In some examples, the level of CpG methylation is measured by a bisulphite sequencing assay, which may involve pyrosequencing.

In any of the methods described herein, the biological sample contains nasal epithelial cells. In some examples, the subject is a human asthma patient who has undergone or is undergoing a steroid treatment.

In some embodiments, any of the methods described herein can further comprise maintaining or repeating the steroid treatment, if the subject is responsive or likely to respond to the steroid treatment. In other embodiments, the method can further comprise applying an alternative treatment to the subject, if the subject is not responsive or not likely to respond to the steroid treatment. The alternative treatment can be a non-steroid treatment, or a combined therapy comprising a non-steroid treatment and a steroid treatment.

In other embodiments, the subject is a human patient free of steroid treatment. The method described herein can further comprise applying a steroid treatment to the subject, if the subject is responsive or likely to respond to the steroid treatment. Alternatively or in addition, the method can further comprise applying a non-steroid treatment or a combined therapy comprising a non-steroid treatment to the subject, if the subject is not responsive or not likely to respond to the steroid treatment.

In some examples, the non-steroid treatment involves a mast cell stabilizer, a leukotriene modifier, an immunomodulator, or a combination thereof. In some examples, the steroid treatment comprises prednisone, corticosteroid, methylprednisolone, dexamethasone, or a combination thereof.

Any of the methods described herein may further comprise monitoring development of an asthma symptom of the subject who is at risk for asthma, if the subject is not responsive or not likely to respond to a steroid treatment. Alternatively or in addition, the method may further comprise performing a home intervention to reduce the risk for asthma development, if the subject is not responsive or not likely to respond to a steroid treatment. In other embodiments, the method may further comprise reducing environmental risk factors for asthma development, if the subject is not responsive or not likely to respond to a steroid treatment.

In another aspect, the present disclosure provides a method of treating a subject with asthma, the method comprising: (a) applying a steroid treatment to a subject in need thereof, wherein the subject exhibits the same or elevated level of VNN1 expression in nasal epithelial cells; or (b) applying a non-steroid treatment or a combined therapy comprising a non-steroid treatment and a steroid treatment to a subject in need thereof, wherein the subject exhibits a decreased level of VNN1 in nasal epithelial cells. In some examples, the subject is a human patient suffering from an asthma exacerbation. In other examples, the subject is a child who is 18 years old or younger.

In some embodiments, the level of VNN1 expression is represented by the level of VNN1 mRNA or the level of CpG methylation in the promoter of a VNN1 gene. In other embodiments,
the level of VNN1 expression is represented by the level of methylation at the CpG4 site in the promoter of the VNN1 gene.

Also within the scope of the present disclosure is a kit for determining a level of VNN1 expression in a biological sample, the kit comprising one or more agents for measuring the level of VNN1 expression. The one or more agents can be: (i) an antibody specifically binding to VNN1 protein;(ii) one or more oligonucleotides, at least one being complementary to a region within the mRNA of VNN1; or (iii) bisulfite and one or more oligonucleotides for use in bisulfite sequencing.

Other aspects of the present disclosure relate to a method of treating a subject with asthma, the method comprising administering an effective amount of cysteamine or a pharmaceutically acceptable salt thereof (e.g., cysteamine bitartrate or cysteamine hydrochloride) to a subject having asthma. The subject (e.g., a human patient) does not respond (fully or partially) to or is not likely to respond to a steroid treatment. In some embodiments, the cysteamine is in disulfide form. In some embodiments, the subject has a decreased level of VNN1 expression relative to a pre-determined value. In some embodiments, the decreased level of VNN1 expression is represented by a decreased level of VNN1 mRNA or a decreased level of VNN1 protein in a biological sample of the subject. In some embodiments, the decreased level of VNN1 expression is represented by a decreased level of CpG methylation in the promoter of a VNN1 gene. In some embodiments, the level of CpG methylation is the methylation level of CpG4 in the promoter of the VNN1 gene.

The present disclosure also provides pharmaceutical compositions for use in treating a subject having asthma and is not responsive (fully or partially) or not likely to respond to a steroid treatment, wherein the pharmaceutical composition comprises cysteamine or a pharmaceutically acceptable salt thereof and a pharmaceutically acceptable carrier. The present disclosure also features uses of cysteamine or a pharmaceutically acceptable salt thereof in manufacturing a medicament for use in treating a subject who has asthma and does not respond to or is unlikely to respond to a steroid treatment.

The details of one or more embodiments of the disclosure are set forth in the description below. Other features or advantages of the present disclosure will be apparent from the following drawings and detailed description of several embodiments, and also from the appended claims.

### BRIEF DESCRIPTION OF THE DRAWINGS

The following drawings form part of the present specification and are included to further demonstrate certain aspects of the present disclosure, which can be better understood by reference to one or more of these drawings in combination with the detailed description of specific embodiments presented herein.

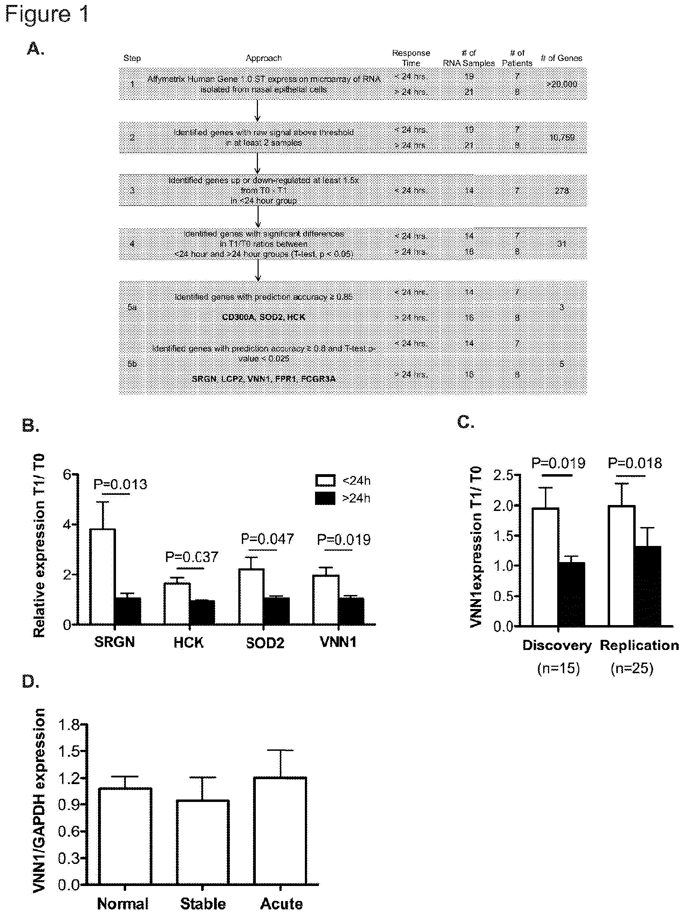

Figure 1 summarizes the analysis of the discovery microarray expression data. Panel A: a diagram of the exemplary methods performed in Example 1. Panel B: a graph showing exemplary relative expression (T₁/T₀) of the genes SRGN, HCK, SOD2, and VNN1 for good responders (<24h, white bars) and poor responders (>24h, black bars). Panel C: a graph showing exemplary VNN1 expression in the discovery cohort and the replication cohort for good responders (<24h, white bars) and poor responders (>24h, black bars). Panel D: a graph showing exemplary VNN1 expression (normalized with GAPDH expression) in children with stable asthma ("stable"), children presenting with an acute asthma exacerbation ("acute"), and non-asthmatic control children ("normal).

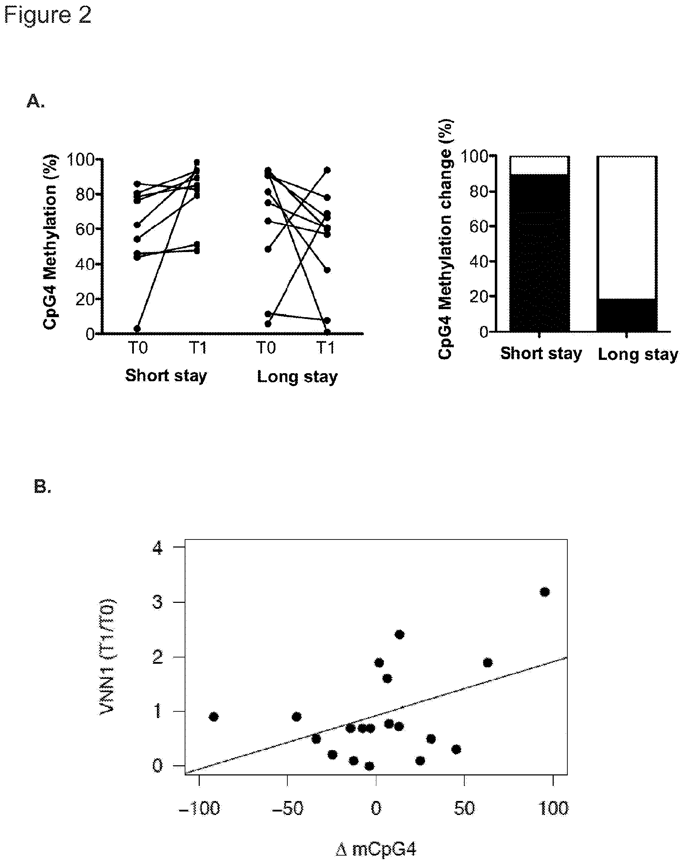

Figure 2 shows differential VNN1 methylation in response to steroid treatment in good versus poor treatment response groups. Panel A: two graphs showing exemplary CpG4 methylation at T₀ and T₁ for good responders (short stay) and poor responders (long stay) and exemplary CpG methylation change for good responders (short stay) and poor responders (long stay). Panel B: a graph showing an exemplary correlation between VNN1 expression and the change in CpG4 methylation.

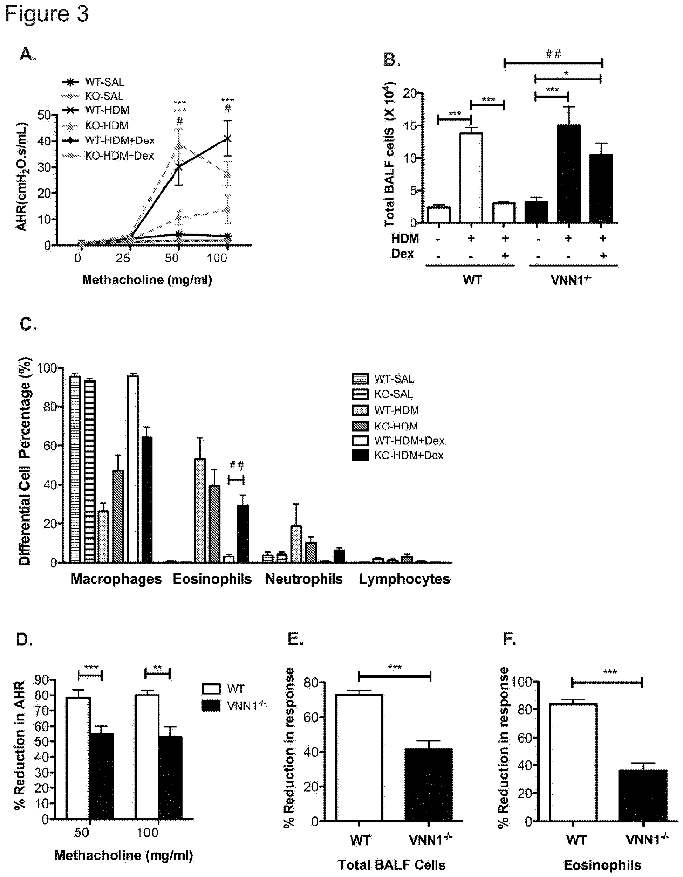

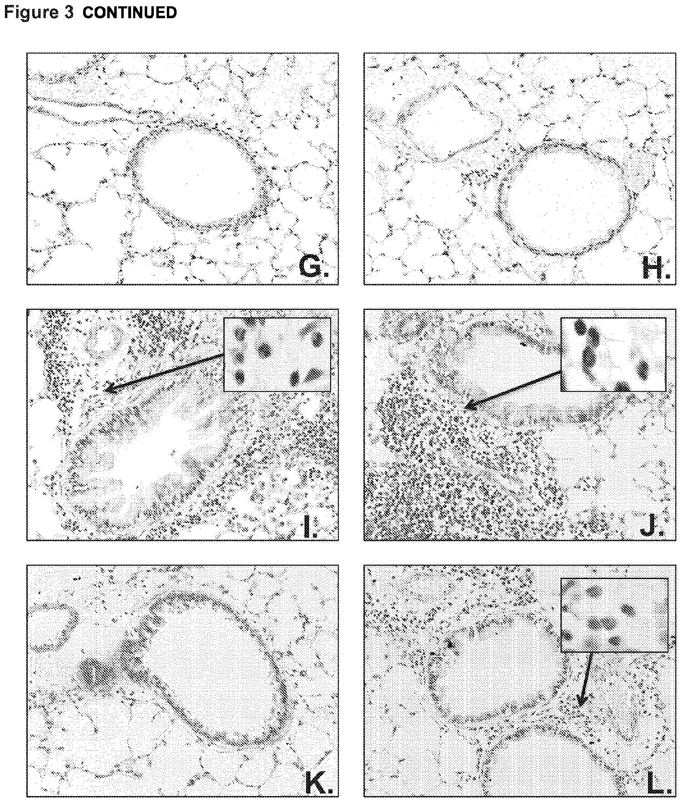

Figure 3 shows repeated house dust mite (HDM) exposure induced allergic airway inflammation and AHR in both WT and VNN1-/- mice, and the phenotype was comparable between two groups. Panel A: a graph showing exemplary airway hyperresponsiveness (AHR) in wild-type mice (WT) or VNN1^{-/-} mice (KO) treated with methacholine in combination with saline (SAL), HDM, or HDM plus dexamethasone (HDM+Dex). Panel B: a graph showing exemplary total bronchoalveolar lavage fluid (BALF) cells in wild-type mice (WT) or VNN1^{-/-} mice treated with saline (SAL), house dust mite (HDM), or HDM plus dexamethasone (Dex). Panel C: a graph showing an exemplary differential cell percentage of macrophages, eosinophils, neutrophils, or lymphocytes in wild-type mice (WT) or VNN1^{-/-}mice (KO) treated with methacholine in combination with saline (SAL), house dust mite (HDM), or HDM plus dexamethasone (HDM+Dex). Panel D: a graph showing exemplary percent reduction in airway hyperresponsiveness (AHR) in wild-type mice (WT) or VNN1^{-/-}mice treated with dexamethasone after exposure to 50 or 100mg/ml methacholine. Panel E: a graph showing exemplary percent reduction in response as indicated by total BALF cells in wild-type mice (WT) or VNN1^{-/-} mice treated with dexamethasone after exposure to methacholine. Panel F: a graph showing exemplary percent reduction in response as indicated by eosinophils in wild-type mice (WT) or VNN1^{-/-} mice treated with dexamethasone after exposure to methacholine. Panels G-L: photos showing airway inflammation in lung tissues of wild-type (WT) and VNN-/- mice treated with dexamethasone.

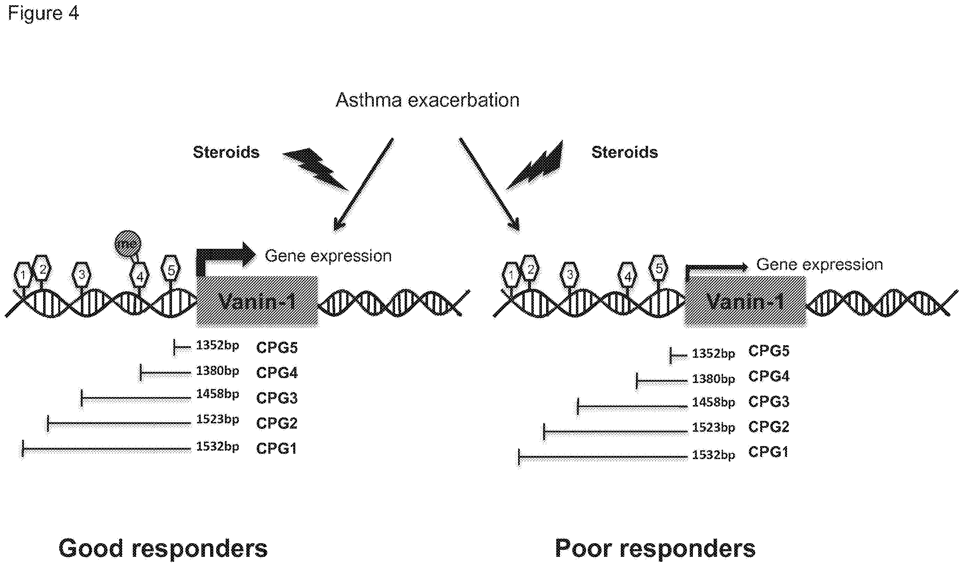

Figure 4 is a diagram showing an exemplary difference in gene expression and methylation in good responders and poor responders.

### DETAILED DESCRIPTION OF THE INVENTION

Asthma affects a large number of people in the US, including children. Although patients suffering from asthma share similar clinical symptoms, the disease is heterogeneous, which contributes to the difficulty in both studying and treating asthma. Children may have poorly controlled asthma for numerous reasons, including lack of compliance with medications, socioeconomic barriers, suboptimal environments with numerous asthma triggers, and biologic causes. It is important to identify the underlying causes that contribute to poorly controlled asthma in each individual so that management strategies can be personalized to achieve the best outcomes. The National Asthma Education and Prevention Program's third Expert Panel emphasizes the importance of individualizing treatment for patients because of the heterogeneous nature of the response to treatment.

As described herein, a study was conducted to identify biomarkers for steroid treatment responsiveness in subjects with asthma. Briefly, children with asthma exacerbation were recruited and followed during hospitalization. Nasal epithelial cells were collected upon presentation to the ED (To) and 18-24 hours later (T₁) and T₁/T₀ gene expression ratios were analyzed to identify genes associated with good and poor treatment response phenotypes. The utility of these genes in discriminating between treatment responsive groups and treatment non-responsive groups was then tested prospectively in a new cohort of patients. A gene candidate (*VNN1*) that consistently discriminated between treatment response phenotypes was further studied in an experimental asthma model and VNN1 promoter methylation was measured by bisulfite pyrosequencing in patients. VNN1 expression changes were associated with treatment response in children with asthma and VNN1 was required for optimal response to steroid treatment an experimental asthma model. A CpG site within the VNN1 promoter was differentially methylated between good versus poor treatment response groups and methylation at this site correlated with VNN1 expression. The results presented herein show that VNN1 contributes to steroid responsiveness and that VNN1 expression correlates with treatment response to steroids in children with asthma. Changes in VNN1 expression were therefore identified as biomarkers of treatment response in subjects with asthma. The results obtained from this study also suggests that VNN1 can be a reliable biomarker for assessing a patient's responsiveness to steroid treatment for other diseases that can be treated by a steroid drug.

Accordingly, described herein are methods for assessing responsiveness to a steroid treatment of a disease that can be treated by steroid (e.g., asthma) in a subject in need thereof based on the expression level of Vanin 1 (VNN1), *e.g.,* the mRNA level of VNN1 and/or the methylation level of one or more CpG sites *(e.g.,* CpG4) in the promoter region of a VNN1 gene. See Figure 4. A suitable treatment can be applied to a subject based on his or her responsiveness to the steroid treatment as determined by the methods described herein. For example, a steroid treatment can be initiated or maintained if the subject is determined as responsive to the steroid treatment. In another example, a non-steroid treatment or a combined treatment comprising a steroid treatment and a non-steroid treatment can be applied to a subject who is determined as not responsive to the steroid treatment as determined by the methods described herein. Also within the scope of this disclosure are kits comprising agents suitable for measuring an expression level of VNN1 in a biological sample, e.g., biological sample containing nasal epithelial cells.

### Methods for Assessing Responsiveness to Steroid Treatment

One aspect of the present disclosure relates to a method of assessing responsiveness to a steroid treatment in a subject, who has, suspected of having, or at risk for a disease (e.g., an inflammatory condition such as asthma) that can be treated by a steroid drug, based on the expression level of VNN1 in a biological sample obtained from that subject. Steroids can be used to treat a variety of conditions associated with malfunctions of the immune system, which cause tissue damages. For example, steroids are used as the main treatment for certain inflammatory conditions, such as systemic vasculitis (inflammation of blood vessels) and myositis (inflammation of muscle or Duchenne's muscular dystrophy). They may also be used selectively to treat autoimmune conditions, such as rheumatoid arthritis, lupus, Sjögren's syndrome, or gout. In addition, steroids such as Prednisolone, Methylprednisolone, and Dexamethasone, are used in cancer treatment.

In some embodiments, the method comprises (a) measuring a level of Vanin 1 (VNN1) expression in a biological sample obtained from a subject having asthma, suspected of having asthma, or at risk for asthma; and (b) assessing the subject's responsiveness to the steroid treatment based on the level of VNN1 expression. In some embodiments, a decreased level of VNN1 expression relative to a pre-determined value indicates that the subject is not responsive or not likely to respond to the steroid treatment. Alternatively or in addition, a same or elevated level of VNN1 expression relative to the pre-determined value indicates that the subject is responsive or likely to respond to the steroid treatment.

### (i) Vanin 1 (VNN1)

Vanins are the only known source of pantetheinase activity in mammalian tissues. VNN-1 is a membrane-associated ectoenzyme with pantetheinase activity that hydrolyzes pantetheine into pantothenic acid (Vitamin B5) and cysteamine. Kaskow et al., Biochemical and biophysical research communications, 2012;417:653-8. In Vanin-1-deficient mice, the lack of detectable tissue cysteamine is associated with an enhanced γ -gluthamylcysteine synthetase activity leading to elevated endogenous glutathione (GSH) stores in tissues. Vanin-1^{-/-} mice display increased resistance to oxidative stress exposure that can be abolished by administration of cystamine (the disulfide form of cysteamine) *(*Mol. Cell. Biol. 24:7214-7224.; J. Clin. Invest. 113:591-597). Enhanced expression of VNN1 has been associated with multiple human diseases, including immune thrombocytopenia (Zhang et al., Blood 2011;117:4569-79), systemic lupus erythematosus (Sanchez-Munoz et al., Lupus, 2013;22:333-5) and inflammatory bowel disease (Gensollen et al., Inflammatory bowel diseases 2013;19:2315-25), although the mechanism for the association is remains unclear. Genetic variants in the VNN1 locus have been linked to IBD susceptibility. *(*Inflamm Bowel Dis. 2013 Oct;19(11):2315-25). See also Berruyer et al., Molecular and cellular biology 2004;24:7214-24.

An exemplary mRNA and protein sequence for human VNN1 are shown below: Human VNN1 mRNA (NM_004666.2)

AGCACTCATTGGACTTCAGCATGACTACTCAGTTGCCAGCTTACGTGGCAATTTTGCTTTTCTATGTCTC
AAGAGCCAGCTGCCAGGACACTTTCACTGCAGCTGTTTATGAGCATGCAGCGATATTGCCCAATGCCACC
CTAACACCAGTGTCTCGTGAGGAGGCTTTGGCATTAATGAATCGGAATCTGGACATTTTGGAAGGAGCGA
TCACATCAGCAGCAGATCAGGGTGCGCATATTATTGTGACTCCAGAAGATGCTATTTATGGCTGGAACTT
CAACAGGGACTCTCTCTACCCATATTTGGAGGACATCCCAGACCCTGAAGTAAACTGGATCCCCTGTAAT
AATCGTAACAGATTTGGCCAGACCCCAGTACAAGAAAGACTCAGCTGCCTGGCCAAGAACAACTCTATCT
ATGTTGTGGCAAATATTGGGGACAAGAAGCCATGCGATACCAGTGATCCTCAGTGTCCCCCTGATGGCCG
TTACCAATACAACACTGATGTGGTATTTGATTCTCAAGGAAAACTGGTGGCACGCTACCATAAGCAAAAC
CTTTTCATGGGTGAAAATCAATTCAATGTACCCAAGGAGCCTGAGATTGTGACTTTCAATACCACCTTTG
GAAGTTTTGGCATTTTCACATGCTTTGATATACTCTTCCATGATCCTGCTGTTACCTTGGTGAAAGATTT
CCACGTGGACACCATAGTATTCCCAACAGCTTGGATGAATGTTTTGCCACATTTGTCAGCTGTTGAATTC
CACTCAGCTTGGGCTATGGGCATGAGGGTCAATTTCCTTGCATCCAACATACATTACCCCTCAAAGAAAA
TGACAGGAAGTGGCATCTATGCACCCAATTCTTCAAGAGCATTTCATTATGATATGAAGACAGAAGAGGG
AAAACTCCTCCTCTCGCAACTGGATTCCCACCCATCCCATTCTGCAGTGGTGAACTGGACTTCCTATGCC
AGCAGTATAGAAGCGCTCTCATCAGGAAACAAGGAATTTAAAGGCACTGTCTTTTTCGATGAATTCACTT
TTGTGAAGCTCACAGGAGTTGCAGGAAATTATACAGTTTGTCAGAAAGATCTCTGCTGTCATTTAAGCTA
CAAAATGTCTGAGAACATACCAAATGAAGTGTACGCTCTAGGGGCATTTGACGGACTGCACACTGTGGAA
GGGCGCTATTATCTACAGATTTGTACCCTGTTGAAATGTAAAACGACTAATTTAAACACTTGCGGTGACT
CAGCTGAAACAGCTTCTACCAGGTTTGAAATGTTCTCCCTCAGTGGCACTTTCGGAACCCAGTATGTCTT
TCCTGAGGTGTTGCTGAGTGAAAATCAGCTTGCACCTGGAGAATTTCAGGTGTCAACTGACGGACGCTTG
TTTAGTCTGAAGCCAACATCCGGACCTGTCTTAACAGTAACTCTGTTTGGGAGGTTGTATGAGAAGGACT
GGGCATCAAATGCTTCATCAGGCCTCACAGCACAAGCAAGAATAATAATGCTAATAGTTATAGCACCTAT
TGTATGCTCATTAAGTTGGTAGAATATTGACTTTTTCTCTTTTTTATTTGGGATAATTTAAAAAATGATG
GATGAGAAAAGAAAGATTGGTCCGGGTTAATATTATCCTCTAGTATAAGTGAATTACTAGTTTCTCTTTA
TTTAGACAAACACACACACACCAGATAATATAAACTTAATAAATTATCTGTTAATGTAGATTTTATTTAA
AAAACTATATTTGAACATTGGTCTTTCTTGGACGTGAGCTAATTATATCAAATAAGTATCACAAATCTTT
TACGCAGAAGAAATAAAAACTACGGGTAGAAAACATAAGAACTATCATAAAATTTACTTACAAGGAGGCT
GCTCTTGTTACCACTTTTATTATATTACGTATCACTTATTCAGCTCTGCTGAAAATTTCCAATGACTTTG
TTTGTTTGCTCTTTTTGTTTTTTACCTAAACAATACATTTTGATTCTCTTGTGGGTTGATAATGTCTCCC
CAAAATTTACATGTTGAAGCACCTCAGAATGTGACTGTATTTGGAGACAGGGTCTTTAAAGAGGTAAAAT
AAGGTCATTAGGATAGACCCTAATTCAATATGACTGATGATCATAAAAGAAGAGGCGAGTAGGGCACAAC
AGGCACAAAGGGAGACCATAAGGAGACACAGAGGAAGGACAACTCTTTACAAGCTAAGAAGAGAGGCCT
CAGAAGAAACCAACCCTGCCAACACCTTGATCTTGGACTTCCAGCCTCCAAAACTATGAGAAATAAATTT
CTATTGTTTAAGTCACCCAGTCCATGGTACTTTGTTAGGCAGCCCTGGCAAATGAATCAAAGACCCATTC
CTGTTCCTCTCCCCACCACTACTGTTTTCTACTGTAATCTGAAGCTTCAACAAAGGCTTACCTGGTAAG
AATATTCAGCTGGTCTGGGTCCTCAAGACTCCAATAGACACTCTTAGAGAAGGATTGCTGATGGATTGAT
AGTGAAACCATTAGATCATTGAATTCCTCTGGAATTAGAAAACCAGAGAGTCCCATTTTAAGAAATTAGA
TATTTAATATAGCATTGTGTGTTCTATTTAGTAACAGCAGAATCTCTTGACATTACACAACTCAGTGAA
ACAACATCATTTAAGCCAAAATATCTCCCAACTGACTGATAGACTCTGAGCACTAATATCATAGTGCTGT
GATGATGGACAATTACATAGTACCGATAACAGCCATGCACTGTGCAAAGCATGCCCTTCTGCACAGGAGA
GCAAGGCACTTGCAGTAGTGATCTATGCCAGCAAAACATCATTTTGAGACAAACATTTTTGTGGCAGATG
TTTTTCCTAAAAAGTACTATATCATCCAAGAAATATTTGAGTAAAATCCCTTGTTCTTTTGGGTGACATT
AACTGACATTTGCTTTTTTTCAAGACCTAATAGAAAATAAGAAAGCCCATAATGTATTTAGAAACAGGAA
TCCTCAGAGCAATTCTCTGTATTCTCATATAATTTCAATGTAAAACAGAAAACATATTGATGTGTTGGTG
ATAGGCTTGAATTATTAAAAACTTCAAAAACATCCTAAGTGTTTCTTTTTTGCTCAACGTTGTCAACTAT
AGTAGGTCTCCCTTGTGGTGTAATGAATTGCCCCCAAACTATTATCTTAAAACAACAAACATTTATTATC
TTATAGCATTTCTGAGGGTCAGGATCTGGGACTGGCTTAGTGGAGTTGTTCTGGATCAGGGCCTTTGGAA
AGTTGTAGTTAACTTGTCCCCAGGGCTGCCATCATCTCAAGGCTCGGGTGGGGCTGGAGAAAATCTGCTT

CTCAGCTCACTCACGGCGGTTGCCAGGCCTCCATTCTTTAGGATGCTAGAAAAACTTTCATAAAATGTCA
TCTGGCTTCTCCTAGAGCAATGATACTGAGAGAGAAAGCACATGAGAGAAAGAGCGAGGGAACTTGGATG
TAAGCCACAGTCTTTGAAAACCTAATCACAGAAGTGACATCTCTTCTTCCACATGATGTTGGTCACATGG
ACCAACAATGGCACAACGTGGACAGAATCAAACAGAGTTGAGAATATCAGGAGGTGGGGCTTCATGGGGG
CCATTTTGGATGCTATCATAGTGAATATATGTATTTATATTTATATCTGTATATATTGCAATGTAATTTA
AAAAATAGGATTGTTTTCCTTTTCTTTTTGCTATATGTGATATGTATTTCAAAATACACTCCCAATAGTT
ACGTCTGAAAAGCACTACACTAAAAAACTTTCTATACATTGAATAATTAAATTAAATAATCTAA (SEQ ID
NO:1)

Human VNN1 protein precursor (NP_004657.2, amino acids 1-21 are a signal peptide, which is cleaved off to form the mature VNN1 protein)

MTTQLPAYVAILLFYVSRASCQDTFTAAVYEHAAILPNATLTPVSREEALALMNRNLDILEGAITSAADQ
GAHIIVTPEDAIYGWNFNRDSLYPYLEDIPDPEVNWIPCNNRNRFGQTPVQERLSCLAKNNSIYVVANIG
DKKPCDTSDPQCPPDGRYQYNTDVVFDSQGKLVARYHKQNLFMGENQFNVPKEPEIVTFNTTFGSFGIFT
CFDILFHDPAVTLVKDFHVDTIVFPTAWMNVLPHLSAVEFHSAWAMGMRVNFLASNIHYPSKKMTGSGIY
APNSSRAFHYDMKTEEGKLLLSQLDSHPSHSAVVNWTSYASSIEALSSGNKEFKGTVFFDEFTFVKLTGV
AGNYTVCQKDLCCHLSYKMSENIPNEVVYALGAFDGLHTVEGRYYLQICTLLKCKTTNLNTCGDSAETAST
RFEMFSLSGTFGTQYVFPEVLLSENQLAPGEFQVSTDGRLFSLKPTSGPVLTVTLFGRLYEKDWASNASS
GLTAQARIIMLIVIAPIVCSLSW (SEQ ID NO:2)

Human isoforms of VNN1 and VNN1 of other species are known in the art and can be retrieved from gene database (e.g., GenBank), e.g., using the above noted human sequences as queries.

### (ii) Measuring VNN1 expression levels

An expression level of VNN1 *(e.g.,* VNN1 protein level, VNN1 mRNA level, or methylation level of one or more of CpG sites in the VNN1 promoter region) in a biological sample of a subject in need of the treatment can be measured via a routine method, e.g., those described herein.

### Subjects

In some embodiments, a subject to be examined by any of the methods described herein can be a mammal, e.g., a human, having, suspected of having, or at risk for asthma. In some embodiments, the subject is a human patient *(e.g.,* a child) suffering from an asthma exacerbation, also known as asthma attack, such as an acute asthma attack. In some embodiments, the subject is a child who is 18 years old or younger, *e.g.,* 5-18 years old, inclusive.

Asthma is an inflammatory disease of the airways. Common symptoms include wheezing, coughing, chest tightness, and shortness of breath. The severity and recurrence of symptoms vary between subjects. Asthma may also be classified as atopic (extrinsic) or nonatopic (intrinsic) where atopy refers to a predisposition toward developing type 1 hypersensitivity reactions. A subject having asthma may be diagnosed based on clinically available tests and/or an assessment of the pattern of symptoms in a subject and response to therapy. An exemplary available diagnostic test for asthma is spirometry. Spirometry is a lung function test that measures the volume and/or flow of air that can be inhaled and exhaled by a subject. Spirometry may be part of a bronchial challenge test, which may involve assessing bronchial hyperresponsiveness to rigorous exercise, inhalation of cold/dry air, and/or a pharmaceutical agent such as methacholine or histamine. Diagnostic methods for asthma are known in the art (see, e.g., Expert Panel Report 3: Guidelines for the Diagnosis and Management of Asthma. NIH Publication Number 08-5846 ed, National Institutes of Health, 2007).

A subject suspected of having asthma may exhibit one or more common symptoms of asthma, such as those indicated above. Such a subject can also be identified by routine medical procedures. A subject at risk for asthma can be associated with one or more risk factors of asthma. Such risk factors include, but not limited to, family history of asthma (e.g., having a blood relative such as a parent or sibling, with asthma), other allergic conditions *(e.g.,* such as atopic dermatitis or allergic rhinitis), overweight, smoking or exposure to secondhand smoke, Exposure to exhaust fumes or other types of pollution, and exposure to occupational triggers, such as chemicals used in farming, hairdressing and manufacturing. Gender and age may also play roles in asthma development. For example, childhood asthma occurs more frequently in boys than in girls.

In some embodiments, the subject is a human patient who is undergoing or has undergone a treatment of asthma, such as a steroid treatment (including those described herein). In other embodiments, the subject is a human patient free of steroid treatment.

### Biological Samples

A suitable biological sample can be obtained from a subject as described herein via routine practice. Non-limiting examples of biological samples include fluid samples such as blood (e.g., whole blood, plasma, serum), urine, and saliva, and solid samples such as tissue *(e.g.,* skin, lung, nasal) and feces. Such samples may be collecting using any method known in the art or described herein, e.g., buccal swab, nasal swab, venipuncture, biopsy, urine collection, or stool collection. In some embodiments, the biological sample comprises nasal epithelial cells. Nasal epithelial cells may be collected, for example, by swabbing inside of a nostril of the subject.

In some embodiments, any of the exemplary samples as described herein (e.g., a biological sample containing nasal epithelial cells) can be obtained from a subject prior to a steroid treatment. In other embodiments, the sample is obtained during the course of a steroid treatment or after the steroid treatment.

In some embodiments, the sample may be processed or stored. Exemplary processing includes, for example, cell lysis and extraction of materials from the lysate (e.g., DNA, RNA, or protein). Exemplary storage includes, e.g., adding preservatives to the sample and/or freezing the sample.

### Determining VNN1 expression levels

The expression level of VNN1 in a biological sample can be represented by the level of VNN1 protein in the sample, the level of VNN1 mRNA in the sample, the methylation level of one or more CpG sites in the promoter region of the VNN1 gene, the activity level *(e.g.,* the pantetheinase enzymatic activity level) of the VNN1 protein, or a combination thereof. Assays for measuring levels of mRNA, protein and CpG methylation are known in the art and described herein, e.g., including probe-based assays, array-based assays, PCR-based assays, bead-based assays, immuno-based assays, sequencing, bisulfate assays, etc. (see, e.g., Molecular Cloning: A Laboratory Manual, J. Sambrook, et al., eds., Fourth Edition, Cold Spring Harbor Laboratory Press, Cold Spring Harbor, New York, 2012; Current Protocols in Molecular Biology, John Wiley & Sons, Inc., New York; Current Protocols in Gene Expression, John Wiley & Sons, Inc., New York; Microarray Methods and Protocols, R. Matson, CRC Press, 2012; Antibodies: A Laboratory Manual, Cold Spring Harbor Laboratory Press, 2nd ed., 2013).

In some examples, the level of VNN1 protein in a biological sample, such as a sample containing nasal epithelial cells, is measured via a suitable method. Exemplary protein level assays include, but are not limited to, immunoassays (e.g., Western blot or enzyme-linked immunosorbent assay (ELISA)) and multiplex bead-based assays. Such assays are known in the art and commercially available.

A VNN1 protein level may be detected using one or more protein binding partners to VNN1. Protein binding partners may be designed using the sequences provided herein or known in the art. In some embodiments, the binding partner is an antibody that is specific for VNN1. As used herein, "specific for" or "specifically binds" refers to the ability of an antibody to preferentially bind to VNN1, with an affinity that is at least two-fold, 10-fold, 50-fold, 100-fold, or better (smaller Kd) than its affinity for binding to a non-specific antigen (e.g., actin, casein) other than VNN1. As used herein, "binding affinity" refers to the apparent association constant or Ka. The Ka is the reciprocal of the dissociation constant (Kd). A binding protein may, for example, have a binding affinity of at least 10⁻⁵, 10⁻⁶, 10⁻⁷, 10⁻⁸, 10⁻⁹, 10⁻¹⁰ and 10⁻¹¹ M for a particular target molecule. Higher affinity binding of a binding ligand to a first target relative to a second target can be indicated by a higher Ka (or a smaller numerical value Kd) for binding the first target than the Ka (or numerical value Kd) for binding the second target. In such cases, the binding protein has specificity for the first target (e.g., a protein in a first conformation or mimic thereof) relative to the second target (e.g., the same protein in a second conformation or mimic thereof; or a second protein). Differences in binding affinity (e.g., for specificity or other comparisons) can be at least 1.5, 2, 3, 4, 5, 10, 15, 20, 50, 70, 80, 100, 500, 1000, or 10⁵ fold.

As used herein, the term "antibody" refers to a protein that includes at least one immunoglobulin variable domain or immunoglobulin variable domain sequence. For example, an antibody can include a heavy (H) chain variable region (abbreviated herein as V_{H}), and a light (L) chain variable region (abbreviated herein as V_{L}). In another example, an antibody includes two heavy (H) chain variable regions and two light (L) chain variable regions. The term "antibody" also encompasses antigen-binding fragments of antibodies (e.g., single chain antibodies, Fab and sFab fragments, F(ab')₂, Fd fragments, Fv fragments, scFv, and dAb fragments) as well as complete antibodies. Methods for making antibodies and antigen-binding fragments are well known in the art (see, e.g. Molecular Cloning: A Laboratory Manual, supra; Lewin's Genes XI, Jones & Bartlett Learning, 11th ed., 2012; Roitt's Essential Immunology, Wiley-Blackwell, 12th Ed., 2011; Current Protocols in Immunology, Wiley Online Library, 2014; WO2006/040153; WO2006/122786; and WO2003/002609).

Binding affinity can be determined by a variety of methods including equilibrium dialysis, equilibrium binding, gel filtration, ELISA, surface plasmon resonance, or spectroscopy (e.g., using a fluorescence assay). Exemplary conditions for evaluating binding affinity are in PBS (phosphate buffered saline) at pH 7.2 at 30°C. These techniques can be used to measure the concentration of bound and free binding protein as a function of binding protein (or target) concentration. The concentration of bound binding protein ([Bound]) is related to the concentration of free binding protein ([Free]) and the concentration of binding sites for the binding protein on the target where (N) is the number of binding sites per target molecule by the following equation: [Bound] = N • [Free]/((1/Ka) + [Free]).

It is not always necessary to make an exact determination of Kₐ, though, since sometimes it is sufficient to obtain a quantitative measurement of affinity, e.g., determined using a method such as ELISA or FACS analysis, is proportional to Ka, and thus can be used for comparisons, such as determining whether a higher affinity is, *e.g.,* 2 fold higher, to obtain a qualitative measurement of affinity, or to obtain an inference of affinity, e.g., by activity in a functional assay, e.g., an in vitro or in vivo assay.

In other examples, a level of VNN1 mRNA is determined in a method described herein.

Exemplary mRNA level assays include, but are not limited to probe-based assays (e.g., northern blots, nuclease protection assays, in situ hybridization), array-based assays (e.g., microarrays), PCR-based assays (e.g., quantitative PCR), multiplex bead-based assays (e.g., commercially-available Luminex^{®} technology such as xMAP^{®} and xTAG^{®}, Illumina), and sequencing-based assays. Such assays are known in the art and commercially available.

An mRNA level of VNN1 may be detected using one or more mRNA binding partners to VNN1 mRNA. mRNA binding partners include oligonucleotides or modified oligonucleotides (e.g., locked nucleic acid oligonucleotides) probes or primers that are complementary to a target mRNA or to a cDNA produced from the target mRNA. Probes and primers may be designed using the sequences provided herein or known in the art. Methods for designing and producing probes and primers are well known in the art and commercially available (see, *e.g.,* US Patent No. 8036835; Rimour et al. GoArrays: highly dynamic and efficient microarray probe design. Bioinformatics (2005) 21 (7): 1094-1103; and Wernersson et al. Probe selection for DNA microarrays using OligoWiz. Nat Protoc. 2007;2(11):2677-91; Primer Design Tool from NCBI: ncbi.nlm.nih.gov/tools/primer-blast/; Premier Biosoft PrimerPlex or Primer Premier).

Complementary, as the term is used in the art, refers to the capacity for precise pairing between two nucleotides. For example, if a nucleotide at a certain position of an oligonucleotide is capable of hydrogen bonding with a nucleotide at the same position of a VNN1 mRNA or cDNA, then the oligonucleotide and the VNN1 mRNA or cDNA are considered to be complementary to each other at that position. The oligonucleotide and the VNN1 mRNA or cDNA are complementary to each other when a sufficient number of corresponding positions in each molecule are occupied by nucleotides that can hydrogen bond with each other through their bases. Thus, "complementary" is a term which is used to indicate a sufficient degree of complementarity or precise pairing such that stable and specific binding occurs between the oligonucleotide and the VNN1 mRNA or cDNA. For example, if a base at one position of an oligonucleotide is capable of hydrogen bonding with a base at the corresponding position of a VNN1 mRNA or cDNA, then the bases are considered to be complementary to each other at that position. 100% complementarity is not required. In some embodiments, the oligonucleotide may be at least 80% complementary to (optionally one of at least 85%, 90%, 91%, 92%, 93%, 94%, 95%, 96%, 97%, 98%, 99% or 100% complementary to) the consecutive or non-consecutive nucleotides of a VNN1 mRNA or cDNA.

In yet other examples, the methylation level of one or more CpG sites in the promoter region of the VNN1 gene is measured in a method described herein. As shown in Figure 4, the VNN1 promoter region comprises at least five CpG sites:
- CpG1: 1532bp upstream from the transcription start site of VNN1;
- CpG2: 1523bp upstream from the transcription start site of VNN1;
- CpG3: 1458bp upstream from the transcription start site of VNN1;
- CpG4: 1380bp upstream from the transcription start site of VNN1;
- CpG5: 1352bp upstream from the transcription start site of VNN1.

The genomic sequence of the VNN1 gene is known in the art. See, e.g., Ensemble ID ENSG0000112299. Provided below is the nucleotide sequence comprising the promoter region of the VNN1 gene, which was obtained from the UCSC genome browser, hg19 assembly. The CpG sites (1-5) are marked in bold and underlined font, with CpG site 1 being the first CpG site marked below, CpG site 2 being the second CpG site marked below, CpG site 3 being the third CpG site marked below, etc.

agcagttagagaagctggtgtttaccggaaactcaggagaaccagccaac
cacaacccctgggttaccttggcaattgcagaataaatgcattatagtta
ctaaagtaaaaaattagatatgcctgtttgcagattgaactataaaaata
ccattcaaagacaaatagatctaaaaataaaatggaaaaacataaacact
aattctgtaaatattatacttaatgcacaactgaaacaaaatttgccagc
ttactcaatatcaaaatctatgaacagtttttctattttatataatttcc
ctctcctctctctggatctcgctccccagctcatttttttcttttttttgc
tctgattctttatacacctctgttgcctctgtgataagcagcttcaaaga
tggttcctaatgctttattggatagaatacaacaaaagcgatgaggtgtt

gcttccccaattacattacgaagcatccgtggcttccatctccagtgggt
CpG1 CpG2
Tcacttgctgtctggctctaagggaatccagataccataatgcgggctgc
CpG3
cctatggtgaggtttgcatcactaggaactcatgtctctgggcaacaacc
aatgaggtcttgatccctgccgtcagccacatgagggagcttggagctcg
CpG4 CpG5
gaagtgaatcctcctggagtcaagccttgatatagctagccctggcagct
gcttgactgcagccttgtgaaagagaccttgggccagaggcaccagctaa
actgcccctggattcctgacccagagaaagtgggagatgatgtattttg
ctttttgaagctgctgaatttggggataatttgttatatagcaatagaaa
atgagtaactcttttgtattcctctttgtcctggcttccccattttgagg
aaaataaagtaaatcaaagtgtagagctgaaatattcacatgaaaataat
aataaagttttaaaattatttgaatgtcttgtgttgacattccaaaatat
atgaattccaaaaatttatatgttgaagtcctaactgtcagtatcttaga
atgtaactttttttggaaaaggggtcatttcagatctaattagttaagatg
aagttatactggagtacagtgggcactaaatcgaattggtcctatgattg
agtctcagtctttcagtgagcctgtaccccctgggtttatgaccttcagtt
ggctttttttcttctgcccttatttggcataaaaacaaagcaggtggatca
cctgaggtcagcaatttgagaccagcctgcccaacacggcgaaaccctat
ctctactaaaaatacaaaaaattagcctggcgtggtggcgggcgcctgta
atcccagctacttgggaggctgaggcaggagaatcacatgaacccgagag
gcggaggttgcagtgagccgagatttcgccactgcactctagcctgggtg
acaagagtgaaactccatctcaaacaacaacaacaataaacaaacaacaa
cgatgacaaaaaaagctagagctgggattttccctttccctgtgttaaag
attagagtggtgtcctcacaaaagggaaaacttggatacaggcacacac
atggggagaatagcatatgaagagacacagggagaaggcagccatctatg
ggtcaaggagagaggcctggaacacatctttccttcaccgccctcaggag
gaaccaactctgctgacaccttcatctgggactcccaccctccagaactg
caaagcaataaatttttatttttacaccacccagtttattgtattttg
ttaggcagccctagcgaactaatgtacatagagttcttgagttaatcttc
acaaattactgcaataaggtagggtcttttgttatgtaacaatgctatga
aatcatagcgttttcttaattaacttccgtagtttaaggtactaagttct
ggacaccacgtgtcttctttctataaataccaggacatgctctgtttttc (SEQ ID NO:
3)

In one example, the methylation level of CpG4 is measured and used for determining the subject's responsiveness to a steroid treatment.

Any conventional method can be used for determining methylation levels of CpG sites in a promoter region. Exemplary CpG methylation assays include, but not limited to bisulfite assays, such as bisulfite sequencing assays and bisulfite PCR assays (see, *e.g.,* EZ DNA Methylation kit^{™} from Zymo Research), and methylation enrichment assays (see, *e.g.,* MethylMiner^{™} Methylated DNA Enrichment Kit from Life Technologies). Bisulphite sequence applies bisulphite treatment of DNA to determine its pattern of methylation. Treatment of DNA with bisulphite converts cytosine residues to uracil but not 5-methycytosine residues, thus introducing specific changes in the DNA sequence that depends on the status of methylation of individual cytosine residues. Such changes indicate the methylation status of a segment DNA, such as a promoter region of a target gene.

An exemplary bisulfite sequencing assay comprises contacting a genomic DNA sample with sodium bisulfite to convert unmethylated cytosines to uracils. The methylated cytosines, which are not converted to uracils, can be detected using sequencing either with or without prior PCR amplification. The type of sequencing performed can be, for example, pyrosequencing, single-molecule real-time sequencing, ion torrent sequencing, sequencing by synthesis, sequencing by ligation (SOLiD^{™}), and chain termination sequencing (e.g., Sanger sequencing). Sequencing methods are known in the art and commercially available (see, *e.g.,* Ronaghi et al.; Uhlén, M; Nyrén, P (1998). "A sequencing method based on real-time pyrophosphate". Science 281 (5375): 363; and Ronaghi et al.; Karamohamed, S; Pettersson, B; Uhlén, M; Nyrén, P (1996). "Real-time DNA sequencing using detection of pyrophosphate release". Analytical Biochemistry 242 (1): 84-9.; and services and products available from Roche (454 platform), Illumina (HiSeq and MiSeq systems), Pacific Biosciences (PACBIO RS II), Life Technologies (Ion Proton^{™} systems and SOLiD^{™} systems)).

Besides pyrosequencing, assays that can be used in bisulphite sequencing include non-methylation-specific PCR based methods, (e.g., direct sequencing, methylation-sensitive single-strand confirmation analysis (MS-SSCA), high resolution melting analysis (HRM), methylation-sensitive single-nucleotide primer extension (MS-SnuPE), base-specific cleavage/MALDI-TOF), methylation-specific PCR (MSP), and microarray-based methods., all of which are known in the art.

In some examples, the activity level of VNN1 protein in a biological sample, such as a sample containing nasal epithelial cells, is measured via a suitable method. Exemplary activity level assays include enzymatic assays such as pantetheinase assays for measuring hydrolysis of pantetheine or a detectable pantothenate derivative. Such assays are known in the art and commercially available (see, *e.g.,* Ruan et al. A fluorescent assay suitable for inhibitor screening and vanin tissue quantification. Anal Biochem. 2010; 399(2):284-92; and Dupre et al. Continuous spectrophotometric assay of pantetheinase activity. Anal Biochem. 1984 Oct;142(1):175-81).

### (iii)Determining responsiveness to steroid treatment of asthma based on VNN1 expression levels

The VNN1 expression level of a biological sample obtained from a subject as described herein can be relied on to determine whether the subject is responsive to a steroid treatment of asthma. In some examples, the VNN1 expression level can be compared with a pre-determined value as described herein. A reduced level of VNN1 expression in the biological sample as compared with the pre-determined value indicates that the subject is responsive or likely to respond to a steroid treatment. Alternative or in addition, the same or elevated level of VNN1 in the biological sample as compared with the pre-determined value indicates that the subject is not responsive or likely not respond to a steroid treatment.

As used herein, "an elevated level of VNN1 expression" means that the level of VNN1 is above a pre-determined value, such as a pre-determined threshold or a control level of VNN1 expression. Control levels are described in detail herein. An elevated level of VNN1 expression includes a VNN1 expression level that is, for example, 1%, 5%, 10%, 20%, 30%, 40%, 50%, 60%, 70%, 80%, 90%, 100%, 150%, 200%, 300%, 400%, 500% or more above a pre-determined value. An elevated level of VNN1 expression also includes increasing a phenomenon from a zero state (e.g., no or undetectable VNN1 expression in a control) to a non-zero state (e.g., some VNN1 expression or detectable VNN1 expression in a sample).

As used herein, "a decreased level of VNN1 expression" means that the level of VNN1 is below a pre-determined value, such as a pre-determined threshold or a control level of VNN1 expression. A decreased level of VNN1 expression includes a VNN1 expression level that is, for example, 1%, 5%, 10%, 20%, 30%, 40%, 50%, 60%, 70%, 80%, 90%, 100%, 150%, 200%, 300%, 400%, 500% or more below a pre-determined value. An elevated level of VNN1 expression also includes decreasing a phenomenon from a non-zero state (e.g., some VNN1 expression or detectable VNN1 expression in a sample) to a zero state (e.g., no or undetectable VNN1 expression in a control).

A pre-determined value can be the VNN1 expression level in a control sample (a controlled level), which can be measured using any of the methods known in the art or described herein. In some examples, the pre-determined value is measured by the same method applied for measuring the VNN1 expression level in a biological sample. The control level may be a level of the VNN1 expression in a control sample, control subject, or a population of control subjects.

The control may be (or may be derived from) a normal subject (or normal subjects). Normal subjects, as used herein, refer to subjects that are apparently healthy and show no signs or symptoms of asthma. The population of control subjects may therefore be a population of normal subjects.

Alternatively, the control sample may be (or may be derived from) a subject or subjects having asthma who is/are responsive to steroid treatment. In some embodiments, the control sample may be (or may be derived from) the subject being assessed for responsiveness to a steroid treatment. For example, the control sample may be a sample derived from the subject prior to steroid treatment.

It is to be understood that the methods provided herein do not require that a control level be measured every time a subject is tested. Rather, in some embodiments, it is contemplated that control levels are obtained and recorded and that any test level is compared to such a pre-determined level. The pre-determined level may be a single-cutoff value or a range of values.

By comparing the VNN1 expression level of a biological sample obtained from a subject and the pre-determined value as described herein, the subject can be identified as responsive or
or likely to be responsive or as not responsive or not likely to be responsive to steroid treatment based on the assessing. In some embodiments, the subject is identified as not responsive or not likely to respond to the steroid treatment if the level of VNN1 expression is a decreased level of VNN1 expression relative to a pre-determined value. In some embodiments, the subject is identified responsive or likely to respond to the steroid treatment if the level of VNN1 expression is the as a same or an elevated level of VNN1 expression relative to the pre-determined value.

### Treatment

In some embodiments, the method further comprises applying a suitable treatment to the subject based on the assessment. If the subject is identified as responsive or likely to respond to the steroid treatment, the method can further comprise maintaining or repeating the steroid treatment. On the other hand, if the subject is identified as not responsive or not likely to respond to the steroid treatment, the method can further comprise applying an alternative treatment to the subject. In some embodiments, the alternative treatment is a combined therapy comprising a non-steroid treatment and a steroid treatment. In other embodiments, the alternative treatment is a non-steroid treatment.

Also within the scope of present disclosure are methods for treating a subject having, suspected of having, or at risk for asthma based on that subject's VNN1 status. In some embodiments, the method comprises applying a steroid treatment to a subject in need thereof, wherein the subject exhibits the same or elevated level of VNN1 expression in nasal epithelial cells. In other embodiments, the method comprises applying a non-steroid treatment or a combined therapy comprising a non-steroid treatment and a steroid treatment to a subject in need thereof, wherein the subject exhibits a decreased level of VNN1 in nasal epithelial cells.

The term "treating" as used herein refers to the application or administration of a steroid treatment, non-steroid treatment, or a combined treatment to a subject, who has asthma, a symptom of asthma, or a predisposition toward asthma, with the purpose to cure, heal, alleviate, relieve, alter, remedy, ameliorate, improve, or affect the disease, the symptoms of the disease, or the predisposition toward the disease.

Exemplary steroid treatment includes, but not limited to, prednisone, corticosteroid, methylprednisolone, dexamethasone, or a combination thereof. In some examples, a steroid treatment is inhaled corticosteroid, including fluticasone (Flovent Diskus, Flovent HFA), budesonide (Pulmicort Flexhaler), mometasone (Asmanex), ciclesonide (Alvesco), flunisolide (Aerobid), and beclomethasone (Qvar). Non-steroid treatment includes, but not limited to, a mast cell stabilizer, a leukotriene modifier, an immunomodulator, a long acting beta agonist, or a combination thereof. Alternatively or in addition, the non-steroid treatment may involve drugs targeting the VNN1 pathway, e.g., vitamin B or a PPARγ agonist. Such non-steroid drugs can enhance the effect of a steroid treatment and thus can be co-used with a steroid treatment. In another example, the non-steroid treatment involves the use of cysteamine, which refers to the compound having the formula HSCH₂CH₂NH₂, or a pharmaceutically acceptable salt thereof (e.g., cysteamine bitartrate or cysteamine hydrochloride). Subjects such as human asthma patients (e.g., child patients) who are identified as not or less likely to be responsive to a steroid treatment can be treated using cysteamine alone, or in combination with another treatment, such as a combination of a steroid drug and cysteamine. In some embodiments, a human asthma patient suitable for cysteamine treatment can be identified by measuring the level of VNN1 expression in a biological sample obtained from the patient. A decreased level of VNN1 expression relative to a pre-determined value as described herein indicates that the patient is suitable for cysteamine treatment. In some embodiments, the level of VNN1 mRNA or the level of VNN1 protein in a biological sample (e.g., nasal cells) of a patient is measured by a conventional method and compared with a predetermined value. In other embodiments, the level of CpG methylation (e.g., the methylation of CpG4 in the promoter of the VNN1 gene) in the promoter region of the VNN1 gene of a patient is determined. A decreased level of the CpG methylation is indicative of a decreased level of VNN1 expression.

In some embodiments, the prednisone is applied by oral administration to the subject and/or the corticosteroid is applied by pulmonary administration. In some embodiments, combined therapy comprises a steroid treatment and a non-steroid treatment as described herein. In some example, a steroid treatment (e.g., prednisone, corticosteroid, methylprednisolone, dexamethasone, or a combination thereof) is co-used with a non-steroid drug that regulates the VNN1 pathway, such as those described herein.

A treatment described herein may be applied in an effective amount. An "effective amount" is that amount of the treatment that alone, or together with further doses, produces the desired response, e.g. elimination or alleviation of symptoms, prevention or reduction the risk of asthma exacerbation, and/or restoration of quality of life. The desired response is to inhibit the progression or the symptoms of the disease. This may involve only slowing the progression of the disease temporarily, although it may involve halting the progression of the disease permanently. This can be monitored by routine methods. The desired response to treatment of the disease also can be delaying the onset or even preventing the onset of the disease.

Such amounts will depend on the particular condition being treated, the severity of the condition, the individual patient parameters including age, physical condition, size, gender and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a patient may insist upon a lower dose or tolerable dose for medical reasons, psychological reasons or for virtually any other reasons.

Administration of a treatment described herein may be accomplished by any method known in the art (see, e.g., Harrison's Principle of Internal Medicine, McGraw Hill Inc., 18th ed., 2011). Administration may be local or systemic. Administration may be, for example, parenteral (e.g., intravenous, subcutaneous, intra-arterial or intradermal), pulmonary (e.g., by inhalation through nose or mouth), or oral. Compositions for different routes of administration are well known in the art (see, e.g., Remington: The Science and Practice of Pharmacy, Pharmaceutical Press, 22nd ed., 2012). The compositions may also be formulated as modified release dosage forms, including delayed-, extended-, prolonged-, sustained-, pulsed-, controlled-, accelerated- and fast-, targeted-, programmed-release, and gastric retention dosage forms. These dosage forms can be prepared according to conventional methods and techniques known to those skilled in the art. Dosage will depend the particular condition being treated, the severity of the condition, the individual patient parameters including age, physical condition, size, gender and weight, the duration of the treatment, the nature of concurrent therapy (if any), the specific route of administration and like factors within the knowledge and expertise of the health practitioner. Dosage can be determined by the skilled artisan.

In some embodiments, the route of administration of the treatment is pulmonary and can be delivered to the lungs by any number of means known in the art. In some embodiments, pulmonary formulations of the present invention are administered as aerosol compositions. Aerosol formulations are known to those skilled in the art and described, for example, in Remington: The Science and Practice of Pharmacy, supra. The aerosol formulation may be, for example, either a solution aerosol, in which the active agents are soluble in the carrier (e.g., propellant), or a dispersion aerosol, in which the active agents are suspended or dispersed throughout the carrier or carriers and optional solvent. In aerosol formulations, the carrier is typically a propellant, usually a liquefied gas or mixture of liquefied gases. For example, the carrier may be a fluorinated hydrocarbon. Exemplary fluorinated hydrocarbons include, but are not limited to, trichloromonofluoromethane, dichlorodifluoromethane, dichlorotetrafluoroethane, chloropentafluoroethane, 1-chloro- 1,1-difluoroethane, 1,1-difluoroethane, octafluorocyclobutane, 1,1,1,2-tetrafluoroethane (HFA-134a), 1,1,1,2,3,3,3-heptafluoropropane (HFA-227) and combinations thereof. As is readily appreciated by one skilled in the art, the aerosol formulations of the invention may include one or more excipients. The aerosol formulations may, for example, contain: a solvent (e.g., water, ethanol and mixtures thereof) for increasing the solubility of the active agent; an antioxidant (e.g., ascorbic acid) for inhibiting oxidative degradation of the active agents; a dispersing agent (e.g., sorbitan trioleate, oleyl alcohol, oleic acid, lecithin, corn oil, and combinations thereof) for preventing agglomeration of particles; and/or a lubricant (e.g,, isopropyl myristate) for providing slippage between particles and lubricating the components, e.g., the valve and spring, of an inhaler. Dry powder formulations for pulmonary delivery include the active agent and any carrier suitable for pulmonary drug administration. The carrier may be, for example, a pharmaceutical sugar such as fructose, galactose, glucose, lactitol, lactose, maltitol, maltose, mannitol, melezitose, myoinositol, palatinite, raffinose, stachyose, sucrose, trehalose, xylitol, hydrates thereof or combinations thereof. Selected components are initially combined and then blended to form a homogeneous, uniform powder mixture. Techniques for preparation of such powders are well known in the art. Regardless of technique employed the resulting powder is preferably both homogeneous and uniform. Typically, the active agents will make up from about 0.10% to about 99% (w/w) of the total formulation.

Pulmonary formulations of the may also be a liquid composition for inhalation, as well known in the art. See, e.g., Remington: The Science and Practice of Pharmacy, supra. Preferably, the liquid is an aqueous suspension, but aqueous solutions may also be used. The liquid formulations may include one or more carriers in addition to the active agents. Generally the carrier is a sodium chloride solution having a concentration making the formulation isotonic relative to normal body fluid. In addition to the carrier, the liquid formulations may contain water and/or excipients including an antimicrobial preservative (e.g., benzalkonium chloride, benzethonium chloride, chlorobutanol, phenylethyl alcohol, thimerosal and combinations thereof), a buffering agent (e.g., citric acid, potassium metaphosphate, potassium phosphate, sodium acetate, sodium citrate, and combinations thereof), a surfactant (e.g., polysorbate 80, sodium lauryl sulfate, sorbitan monopalmitate and combinations thereof), and/or a suspending agent (e.g., agar, bentonite, microcrystalline cellulose, sodium carboxymethylcellulose, hydroxypropyl methylcellulose, tragacanth, veegum and combinations thereof). Combining the components followed by conventional mixing effects a liquid formulation suitable for inhalation. Typically, the active agents will make up from about 0.01% to about 40% of the total formulation.

Various known devices may be used to administer pulmonary formulations, whether dry powder, aerosol or liquid. Dry powder inhalers are well known to those skilled in the art and are used to administer the aforementioned dry powder formulations: Suitable dry powder inhalation devices for administering the present formulations include, for example, TTJRBOHALER^{®} (Astra Pharmaceutical Products, Inc., Westborough, MA), ROTAHALER^{®} (Allen & Hanburys, Ltd., London, England). Aerosol formulations may be administered via pressurized metered-dose inhalers. A metered-dose inhaler may automatically dispense, in a puff intended for inhalation in a single or multiple breaths, a set amount of a treatment described herein when activated by the subject in need of treatment. Liquid formulations of the invention may be administered via a pump spray bottle or nebulizer.

In some embodiments, the route of administration of the treatment is oral. As used herein, oral administration also includes buccal, lingual, and sublingual administration. In some embodiments, treatments provided herein may be provided in solid, semisolid, or liquid composition for oral administration. Suitable oral dosage forms include, but are not limited to, tablets, capsules, pills, troches, lozenges, pastilles, cachets, pellets, medicated chewing gum, granules, bulk powders, effervescent or non-effervescent powders or granules, solutions, emulsions, suspensions, solutions, wafers, sprinkles, elixirs, and syrups. In addition to the active ingredient(s), the compositions may contain one or more pharmaceutically acceptable carriers or excipients, including, but not limited to, binders, fillers, diluents, disintegrants, wetting agents, lubricants, glidants, coloring agents, dye-migration inhibitors, sweetening agents, and flavoring agents.

Preparations for parenteral administration include sterile aqueous or non-aqueous solutions, suspensions, and emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also contain one or more of a preserving agent, a wetting agent, an emulsifying agent and a dispersing agent. The dosage forms may be sterilized by, for example, filtration of the composition, by irradiating the composition, or by heating the composition. They can also be manufactured using sterile water, or some other sterile injectable medium, prior to use.

In some embodiments, the method further comprises taking actions other than or in addition to a treatment based on the assessment as described herein. In some embodiments, the method further comprises monitoring development of an asthma symptom of the subject who is at risk for asthma, if the subject is not responsive or not likely to respond to a steroid treatment. The monitoring may comprise a physical examination and/or spirometry.

In some embodiments, the method further comprises performing a home intervention to reduce the risk for asthma development, if the subject is not responsive or not likely to respond to a steroid treatment. Home intervention may involve reduce the level of exposure to certain matters that may induce asthma, e.g., mold, allergen, etc. In other embodiments, home intervention may involve dietary intervention to regulate the VNN1 pathway or its downstream pathway (e.g., the PPARy pathway). Such dietary intervention can include adding fatty acids such as linoleic acid to diet.

In some embodiments, the method further comprises reducing environmental risk factors for asthma development, if the subject is not responsive or not likely to respond to a steroid treatment. Environmental risk factors refer to those that are likely to induce or enhance asthma. Examples include, but are not limited to, traffic pollution, allergens (e.g., pet allergens such as those from cat, dog, dust mite, pollen), smoke/tobacco exposure, mold exposure, ozone exposure, or NO₂ exposure.

### Kits

Another aspect of the present disclosure relates to kits for use in determining a level of VNN1 expression in a biological sample. Accordingly, in some embodiments, such a kit can comprise reagents for determining a level of VNN1 expression for use in a method described herein.

In some embodiments, the kit comprises one or more agents for measuring the level of VNN1 expression, wherein the one or more agents are: (i) an antibody specifically binding to VNN1 protein; (ii) one or more oligonucleotides, at least one being complementary to a region within the mRNA of VNN1; or (iii) sodium bisulfite and one or more oligonucleotides for use in bisulfite sequencing (e.g., oligonucleotides complementary to a region of VNN1 coding sequence or complementary to a sequence derived from treating a VNN1 coding sequence with bisulfite). The kit may further comprise one or more containers for containing the one or more agents (e.g., tubes or bottles). The kit may further comprise containers for biological samples (e.g., tubes, vials, or bottles).

The antibody specifically binding to VNN1 protein, may be, *e.g.,* a monoclonal or polyclonal antibody. VNN1 antibodies are known in the art or can be designed and produced using methods known in the art (see, *e.g.,* Catalog numbers ab96171 and ab69844 from Abcam^{®}).

In some embodiments, the at least one oligonucleotide being complementary to a region within the mRNA of VNN1 is an oligonucleotide that is complementary to a sequence provided herein (e.g., human VNN1 mRNA sequence as described herein). In some embodiments, the kit further comprises one or more reagents for detecting VNN1 mRNA by PCR (e.g., quantitative PCR), such as one or more of DNA polymerase, dNTPs, a fluorescent dye that binds to DNA *(e.g.,* a cyanine dye such as SYBR^{®} Green from Life Technologies Corp.), and a PCR buffer.

In some embodiments, the oligonucleotides for use in bisulfite sequencing are oligonucleotides that are complementary to a sequence within a VNN1 promoter region, particularly regions upstream and downstream of a CpG site within the VNN1 promoter. In some embodiments, the kit further comprises one or more reagents for bisulfite sequencing (e.g., pyrosequencing), such as one or more of DNA polymerase, ATP sulfurylase, luciferase, apyrase, adenosine 5' phosphosulfate (APS), and luciferin.

In some embodiments, the kit can further comprise an instruction manual providing guidance for using the kit to perform a method described herein. Such instructions may be paper instructions or on an electronic storage medium.

Without further elaboration, it is believed that one skilled in the art can, based on the above description, utilize the present disclosure to its fullest extent. The following specific embodiments are, therefore, to be construed as merely illustrative, and not limitative of the remainder of the disclosure in any way whatsoever. All publications cited herein are incorporated by reference for the purposes or subject matter referenced herein.

### Assessment of Responsiveness to Cysteamine Treatment in Huntington's Disease Using VNN1 as a Biomarker

In another aspect, the present disclosure features a method for assessing whether a subject having, suspected of having, or at risk for Huntington's Disease (HD) would be responsive to cysteamine treatment. To practice this method, a biological sample can be obtained from a candidate subject before, after, or during the course of cysteamine treatment. In some examples, the candidate subject can be a human patient who is suffering from, suspected of having, or at risk for HD. The expression level of VNN1 in the biological sample (e.g., any of those described herein) can be determined via a conventional method or any assay described herein. The expression level of VNN1 can be represented by the level of VNN1 mRNA, the level of VNN1 protein, and/or the level of VNN1 activity such as its enzymatic activity in the biological sample. Alternatively or in addition, the expression level of VNN1 can be represented by the level of methylation at one or more of the CpG sites within the VNN1 promoter region *(e.g.,* the methylation level at CpG4). See above descriptions. The VNN1 level thus obtained may be compared with a pre-determined value to determine whether the candidate subject is or is likely to respond to the cysteamine treatment.

A pre-determined value can be the VNN1 expression level in a control sample (a controlled level), which can be measured using any of the methods known in the art or described herein. In some examples, the pre-determined value is measured by the same method applied for measuring the VNN1 expression level in a biological sample. The control level may be a level of the VNN1 expression in a control sample, control subject, or a population of control subjects.

The control may be (or may be derived from) a normal subject (or normal subjects). Normal subjects, as used herein, refer to subjects that are apparently healthy and show no signs or symptoms of HD. The population of control subjects may therefore be a population of normal subjects.

Alternatively, the control sample may be (or may be derived from) a subject or subjects having HD who is/are responsive to cysteamine treatment. In some embodiments, the control sample may be (or may be derived from) the subject being assessed for responsiveness to cysteamine treatment. For example, the control sample may be a sample derived from the subject prior to the cysteamine treatment.

If the VNN1 level of the biological sample is lower than the pre-determined level *(e.g.,* a lower VNN1 mRNA or protein level, a lower VNN1 activity level, or a lower methylation level at one or more of the CpG sites, such as CpG4 in the VNN1 promoter region), the subject can be identified as a poor responder to the cysteamine treatment. On the other hand, if the VNN1 level of the biological sample is the same or higher than the pre-determined level *(e.g.,* a same or higher VNN1 mRNA or protein level, a same or higher VNN1 activity level, or a same or higher methylation level at one or more of the CpG sites, such as CpG4 in the VNN1 promoter region), the subject can be identified as a good responder to the cysteamine treatment.

When a subject has been assessed for his or her responsiveness to cysteamine, an appropriate treatment can be determined according to the assessment results. For example, if a subject is identified as responsive or likely to be responsive to cysteamine, a cysteamine treatment can be initiated or maintained for that subject. Alternatively, if a subject is identified as not responsive or not likely to be responsive to cysteamine, an alternative treatment can be applied to that subject. In some examples, the alternative treatment is a non-cysteamine treatment, e.g., manoamine inhibitors such as Tetrabenazine (Xenazine); anticonvulsants such as Valproic acid (Depakote, Depakene, or Depacon), or Clonazepam (Klonopin); antipsychotic agents such as Risperidone (Risperdal) or Haloperidol (Haldol); Rausolfia alkaloids such as Reserpine; and antidepressants such as Paroxetine (Paxil). In other examples, the alternative treatment is a combination of cysteamine and a non-cysteamine drug, *e.g.,* those described herein.

### Examples: Nasal Epithelial VNN-1 expression and promoter methylation discriminate asthma treatment response phenotypes in children

Nasal epithelial cells can be readily sampled safely during an asthma attack (Tantisira et al., 2011) and reflect changes observed in the bronchial airways of asthmatic children (Poole et al., J Allergy Clin Immunol., 2014;133:670-8 e12). In the present study, a genomewide expression profiling of nasal epithelial cells was to identify genes whose temporal expression patterns (before and after treatment) consistently and reliably discriminated between treatment response groups among children hospitalized for asthma exacerbation. The potential value of the identified genes was further tested in a replication cohort and in mechanistic studies.

### Methods

### Subjects

Children aged 5-18 years with asthma exacerbation were recruited in this study. Exclusion criteria were: (1) use of oral, nasal, or IV steroids as well as nebulized or inhaled steroids with a face mask within the past 14 days; (2) nasal malformations, tumors, or nasal obstruction that precludes sampling; (3) bleeding diathesis; (4) co-morbid lung condition; (5) history of discharge home after birth from the NICU or nursery on supplemental oxygen; (6) dependence on oral steroids or an immunosuppressive agent for a medical condition other than asthma; or (7) history of a congenital cardiac anomaly and/or heart lesion requiring medication or surgery. Initially, 57 subjects were consented and 15 of these were hospitalized for asthma exacerbation. These 15 patients were used as a discovery cohort to test the association between gene expression and steroid treatment response. To further validate the findings from the discovery cohort, a replication cohort of 25 children hospitalized for asthma were recruited. Twenty children hospitalized for asthma exacerbation were recruited for the methylation studies. Six participants were overlapping between the replication and methylation cohorts.

All subjects provided demographic, environmental, asthma trigger information, and personal and family allergy and asthma history data using study specific questionnaires. To assess baseline asthma symptom severity, a respiratory symptom score (comprised of symptom frequency questions for wheeze, cough, shortness of breath, and chest tightness) was calculated. Butsch Kovacic et al., Pediatr Allergy Immunol Pulmonol 2012;25:104-13. To assess asthma control, the age-specific Asthma Control Test^{™} score was collected on all participants. Liu et al., J Allergy Clin Immunol 2010;126:267-73, 73 e1.

### Treatment protocol

All enrolled patients were placed on the evidence-based treatment protocol for inpatient asthma exacerbation. The admitting physician determined the initial interval of albuterol treatments, which were subsequently spaced based on physician and/or respiratory therapist assessments. All patients received 2mg/kg/day of prednisone while hospitalized and inhaled corticosteroids were continued via mouthpiece. Patients were discharged home when three components were met: (1) oxygen saturations greater than or equal to 91% on room air for at least 6 hours; (2) no evidence of respiratory distress; and (3) successfully receive albuterol nebulizer treatments every 4 hours x 2.

### Good and Poor Treatment Responder Definitions

Length of stay (LOS) was calculated as the time the disposition was set to admit to the time the subject met the clinical criteria for discharge. Good responders were defined as those with LOS<24 hours (short LOS) and poor responders as those with LOS>24 hours (long LOS).

### Nasal epithelial cell sample collection and processing

Nasal epithelial samples were collected at two time points from each subject: (1) in the emergency department (ED) prior to steroid treatment and (2) on the inpatient floor 18-24 hours after receiving steroids in the ED. The procedure, characterization of cell types, sample processing, and RNA isolation have been described previously in detail. See, e.g., Guajardo et al., J Allergy Clin Immunol., 2005;115:243-51. RNA was extracted and submitted to the Center for Environmental Genomics Facility Core (Integrative Technologies Support Core) for gene profiling on the Affymetrix Human Gene 1.0 ST expression array platform. Manual cell differential count was performed using 5 high-power fields and the relative percentage of nasal epithelial cells was calculated. All nasal samples contained >90% epithelial cells, similar to previous findings. Guajardo et al., 2005.

### Microarray Data Analysis

Microarray cell image files were analyzed by using GeneSpring GX software (Agilent Technologies, Santa Clara, CA). Probe level measurements were subject to initial background correction and normalization using the GC-RMA algorithm. The resulting estimated transcript levels were then normalized per chip to the 50^{th} percentile, and per gene to the median intensity. Next, to retain only genes reliably expressed in the nasal cells, the set of genes was further filtered by selecting probe sets where the raw intensity was higher than a threshold of 100 in at least 2 chips.

### Quantitative real time-PCR analyses

Gene-specific primers were designed by using Primer-BLAST software (National Center for Biotechnology Information, NCBI), in which at least one intron was spanned in the genomic sequence to ensure that mRNA-derived products were amplified and the contamination of genomic products was minimized. The sequences of primers for the target genes are listed in Table 1. One to two µg of total RNA was used for cDNA synthesis per sample (SuperScript II cDNA synthesis kit, Invitrogen). RT-PCR analysis was conducted with the iCycler (Bio-Rad, Hercules, CA) by using the iQ SYBR Green Supermix Taq polymerase mix (Bio-Rad). The amount of double-stranded DNA product was indicated by the intensity of SYBR Green fluorescence and measured at the end of each extension cycle. The results were expressed as average fold changes in gene expression relative to the housekeeping gene GAPDH.

**Table 1. Primer sequences used for qRT-PCR testing mRNA expression level of target genes in nasal epithelial cells**

| **Gene** | **ID** | **Sense** | **Anti-sense** |
|---|---|---|---|
| SRGN | NM_002727 | CCTGGTTCTGGAATCCT CA (SEQ ID NO: 4) | TCGAACATTGGTCCTTTTT CTT (SEQ ID NO: 5) |
| SOD2 | NM_001024465 | TTACAGCCCAGATAGCT CTT (SEQ ID NO: 6) | ATGGCTTCCAGCAACTC (SEQ ID NO: 7) |
| HCK | NM_001172133 | TCTGCATCCCTGGTGTG TAA (SEQ ID NO: 8) | AAGTTGATGGCTTCAGGA G (SEQ ID NO: 9) |
| VNN1 | NM_004666 | CTCAGTGGCACTTTCGG (SEQ ID NO: 10) | CAACCTCCCAAACAGAGT TAC (SEQ ID NO: 11) |
| GAPDH | NM_002046 | GGGGAAGGTGAAGGTC GGAGTCA (SEQ ID NO: 12) | AGCCTTGACGGTGCCATG GAAT (SEQ ID NO: 13) |

### DNA isolation, bisulphite treatment, and pyrosequencing analysis

Genomic DNA was extracted from nasal epithelial samples using the Allprep DNA/RNA Micro Kit (Qiagen) according to the manufacturer's protocol. 200ng DNA from each sample was bisulphite modified using the EZ DNA Methylation Kit (Zymo Research). For pyrosequencing, a two-step PCR reaction was performed using primer pairs designed to amplify the target region specifically, with the reverse primer biotinylated. Primer sequences used for the bisulphite pyrosequencing reactions are listed in Table 2. The chromosomal coordinates in the University of California at Santa Cruz February 2009 human genome assembly for each CpG site were shown. The annealing temperature used for both PCR reactions was 50°C. Pyrosequencing analysis was conducted using a Pyromark Q96 MD (Qiagen) and the DNA methylation percentage was determined by Qiagen Pyromark CpG software 1.0.11 (Qiagen).

**Table 2. Primer sequences used for bisulphite pyrosequencing VNN1 DNA methylation measurement in nasal epithelial cells**

| | | | |
|---|---|---|---|
| **PCR** | | **Sense** | **Antisense** |
| | Internal | AGGTGTTGTTTTTTTAATTAT ATTA (SEQ ID NO: 14) | ᵃ5Biosg/CTTAACTCCAAAA AAATTCACTTCC (SEQ ID NO: 15) |
| | External | TTTAAAGATGGTTTTTAATG TTTTATTG (SEQ ID NO: 16) | CCCAAAATCTCTTTCACA AAACTAC (SEQ ID NO: 17) |
| | | | |
| **Sequencing** | | **Chromosomal coordinates^{b}** | **Sequence** |
| | CpG1, 2 | chr6: 133036726 and 133036717 | AGGTGTTGTTTTTTTAATT ATATTA (SEQ ID NO: 18) |
| | CpG3 | chr6: 133036652 | GTTGTTTGGTTTTAAGGG AATTTAG (SEQ ID NO: 19) |
| | CpG4, 5 | chr6: 133036574 and 133036546 | TTGGGTAATAATTAATGA GGTTTTG (SEQ ID NO: 20) |

| | | | |
|---|---|---|---|
| a: 5'-biotin b: The chromosomal coordinates of each CpG site were retrieved from University of California at Santa Cruz February 2009 human genome assembly. | | | |

### Experimental Asthma Model

The VNN1^{-/-} mice used in this study were described in Pitari et al., FEBS letters 2000;483:149-54. Age- and sex-matched wild-type BALB/c mice were purchased from Harlan Laboratories (Indianapolis, IN). All mice were housed in a specific pathogen-free environment following routine practice.

Mice were exposed to intranasal doses of HDM (20 ug in 50 ul saline) or saline (0.9% NaCI, 50 ul; control group) 3 times a week for 3 weeks as previously described18. Mice were treated with intra-peritoneal dexamethasone (3mg/kg in dimethyl sulfoxide, (DMSO)) or DMSO (100 ul) for the last 5 days of the 3 week model. Airway hyperresponsiveness (AHR) was assessed 24 hours after the last HDM challenge using a flexiVent system (SCIREQ, Montreal, QC, Canada) as previously described. See, e.g., Brandt et al., J Allergy Clin Immunol., 2013;132:1194-204 e2. Bronchoalveolar lavage fluid (BALF) was collected, processed, and inflammatory cells were quantified analyzed as previously described. See Brandt et al., 2013.

### Statistical Analysis

To identify gene candidates whose expression level changes following steroid treatment were associated with poor versus good responder phenotypes, a two-phase approach was used: discovery and replication. In the discovery phase, t-tests were first used to validate the results of the microarray data, however, it was apparent that the length of stay was confounded with the time of admission. As some genes may exhibit circadian rhythms in expression, it was important to account for this possible source of bias. Thus, biasreduction logistic regression model was employed to fit the data and calculate the expected probability of being a long stay as proposed by Firth. Firth D., Biometrika 1993;80:27-38. Firth's approach to logistic regression is an improvement over traditional maximum likelihood, and significantly reduces the sample bias even in small samples.

For the replication set, it was first examined whether there were differences between the discovery and replication cohort that may introduce bias. It was found that the time of admission for the discovery cohort was significantly different than the time of admission for the replication cohort. Thus, the replication cohort was matched with discovery cohort based on month, T₀ and T₁ time using propensity scores and individuals were selected from a pool of potential replication samples to be considered. Austin PC. Multivariate behavioral research 2011;46:399-424. Importantly, the gene expression profiles were not considered in this matching process. After matching, t-tests were performed comparing the qPCR results from short and long stay participants. Further, bias reduction logistic regression was employed to ensure that associations were not due to confounding factors.

For the experimental asthma model results, individual AHR, total BALF cell counts and eosinophil percentage in mice treated with HDM plus dexamethasone were compared to and normalized by the corresponding mean value in the HDM-treated group. The data are presented as means ± SEM. Difference between the WT and VNN1^{-/-} group was tested by non-parametric Mann Whitney test. A p-value < 0.05 was considered significant. Percentage reduction was used to present the steroid response results.

For the methylation analysis, the Pearson test was used to analyze the correlation between changes in mRNA expression (T₁/T₀) and DNA methylation (mTi-mTo) of VNN1. Fisher's exact test was used to compare the difference in VNN1 DNA methylation between patients with good treatment response (short stay) and poor treatment response (long stay). A p-value < 0.05 was considered significant.

### Results

### Subjects

The discovery and replication cohorts were primarily male and non-white (Table 3). The discovery cohort was slightly older than the replication cohort, but within each cohort there was no difference in age between the good (<24 hr) and poor (>24 hr) treatment responder groups. There were also no differences in individual parental reported asthma triggers between the discovery and replication cohorts. By design, the discovery and replication cohorts were similar with respect to month admitted, To time, and T₁ time (Table 3). Across all cohorts, there were no differences in baseline asthma symptoms or asthma control scores between the good and poor responders.

**Table 3. Description of Discovery and Replication Cohorts**

| **Parameter** | **Discovery (n=15)** | **Replication (n=25)** | **P-value** | **Methylation (N=20)** |
|---|---|---|---|---|
| Age (mean±sd) | 13.4±3.8 | 8.5±3.3 | 0.00014 | 8.8±4.2 |
| Age range | (7.4, 18.0) | (5.0,17.1) | | (5.0,18.4) |
| Race (white %) | 33.3% | 12.0% | 0.20 | 10.0% |
| Sex (Male %) | 73.3% | 72.0% | 0.78 | 70.0% |
| Month of admission | (4,12) | (3,11) | | (2,12) |
| To time (24 hour clock) | (9.3,20.8) | (10.0,21.4) | | (10.3,18.3) |
| T₁ time (24 hour clock) | (8.1,16.7) | (8.5,17.3) | | (7.7, 18.9) |

### Identification of genes differentially expressed between good and poor responder groups in discovery cohort

Fig. 1A summarizes the analysis of the discovery microarray expression data. There were >20,000 genes on the microarray (Step 1). To identify candidate genes, the following filtering was performed: Step 2: genes exhibiting a raw signal above threshold in at least 2 samples (n=10759); Step 3: genes responsive to treatment (n=278); and Step 4: genes with significant differences (p<0.05) in T₁/T₀ ratio between the good and poor responder groups (n=31). Next, a linear discriminant analysis was applied to the expression data (using the "Ida" function from the MASS package in R). By using leave-one-out cross-validation, the prediction accuracy for each of these 31 genes was calculated to predict whether a given child would be in the <24 or >24 group. Using this approach, there were 8 genes with an estimated prediction accuracy of >0.80 (Step 5). To validate these data, qRT-PCR was performed. Expression of SOD2, HCK, SRGN, and VNN1 was significantly induced in the <24 hr compared to the >24 hr group, validating the array data (Fig. 1B). CD300A was not detectable in most of the nasal samples and reliable results could not be achieved for LCP2, FPR1, and FCGR3A, due to low copy numbers.

### VNN1 expression change predicts steroid treatment response in the replication cohort

In order to substantiate these findings, an independent prospective cohort was recruited to serve as a replication. VNN1 was again downregulated in the >24 hr poor responder group (p=0.018, Fig. 1C), replicating the findings from the discovery cohort (p=0.019, Figure 1C). Expression of SOD2, HCK, and SRGN was not significantly different between the treatment response groups.

In order to evaluate whether the observed change in VNN1 expression before and after treatment was attributable to a baseline difference in VNN1 expression at To, VNN1 expression at To was compared between all patients; no significant difference was detected. To examine whether VNN1 expression was increased during exacerbation relative to stable asthma, data was utilized from a previously published study examining nasal airway gene expression in children with stable asthma (no exacerbation for 6 months), children presenting with an acute asthma exacerbation, and non-asthmatic control children. Guajardo et al., 2005. VNN1 nasal airway expression did not differ among these 3 groups (Fig. 1D). Thus, dysregulation of VNN1 expression does not distinguish between asthma and non-asthma, nor between stable and acute asthma, but rather distinguishes good and poor treatment response among hospitalized asthmatics.

### Differential VNN1 methylation in response to steroid treatment in good versus poor treatment response groups

Since temporal changes in VNN1 expression were significantly associated with treatment response phenotypes in hospitalized asthmatic children and DNA methylation is one of the major epigenetic mechanisms that regulates gene expression, it was hypothesized that steroid treatment may result in differential methylation of VNN1. To test this, the methylation level of 5 CpG sites within the VNN1 promoter (defined as 2kb upstream from the transcription start site) was examined in 20 patients (Table 3) with simultaneous nasal epithelial RNA and DNA samples collected at both time points (T₀ and T₁). The methylation level at the CpG4 site significantly decreased in the poor responders, but increased in good responders following treatment (p=0.005, odds ratio=27.3, Fig. 2A). Further, there was a positive correlation between the change in DNA methylation at CpG4 (mTi-mTo) and VNN1 gene expression (p=0.046, Pearson r=0.46, Fig. 2B). These findings collectively suggest that methylation at the CpG4 site at the promoter of VNN1 might be a crucial molecular event that regulates VNN1 gene expression and modulates host response to steroid treatment.

### VNN^{-/}⁻ mice are resistant to dexamethasone treatment in an experimental asthma model

In order to more directly examine the role of VNN1 in the development of asthma and in the response to treatment for asthma, VNN1^{-/-} mice were studied in an experimental asthma model. Repeated HDM exposure induced allergic airway inflammation and AHR in both WT and VNN1^{-/-} mice, and the phenotype was comparable between two groups (Figs. 3A-C), supporting that VNN1 is not needed for development of the asthma phenotype. In contrast, VNN1 was required for optimal response to steroid treatment in the experimental asthma model. Dexamethasone significantly reduced AHR in the WT mice, with an average percentage reduction of 78.1% (± 5.5%) and 80.2% (± 3.1%) at 50mg/ml and 100mg/ml methacholine challenge, respectively (Fig 3D). Dexamethasone also significantly alleviated airway inflammation in the WT mice (Figs. 3G, I, K), which was reflected by a large reduction in total BALF cells (70.0 ± 4.3%) (Fig. 3E) and eosinophils (83.6 ± 3.7%) (Fig. 3F). In contrast, VNN^{-/-} mice were significantly less responsive to dexamethasone. AHR was reduced by an average percentage of 55.2% (55.2 ± 4.9%) at 50mg/ml methacoline and 53.3% (± 6.5%) at 100mg/ml methacoline (Fig. 3D). Total BALF cells and eosinophils were only reduced by 40.9% (± 4.9%) (Fig. 3E) and 36.1% (± 5.4%) (Fig. 3F), respectively (P<0.001). Consistently, significant numbers of residual eosinophils were still present in the lung tissue after dexamethasone treatment (Fig 3H, J, L) in VNN^{-/-} mice.

### Discussion

The findings obtained from this study demonstrate that nasal epithelial expression of VNN1 and methylation at the CpG4 site of the VNN1 promoter may be useful biomarkers of treatment response phenotypes in asthma patient, such as children hospitalized for asthma exacerbation. Further, these studies demonstrate that VNN1 contributes to optimal steroid treatment response in an experimental asthma model. Children hospitalized for asthma were the subject studied here, because this inpatient setting provides a unique opportunity to characterize response to standardized treatment regimens. Since non-adherence to medications was not an issue during the period that the subject was hospitalized, differences between subjects could be largely attributed to variation in individual host response to treatment. Although our findings stem from the hospital environment, they have broad implications for difficult-to-treat patients who are not fully responsive to steroid treatment and, as a consequence, do not easily achieve asthma control. Bousquet et al., The Journal of allergy and clinical immunology 2010;126:926-38. This group of patients that is the most challenging and accounts for >50% of health care utilization related to this disease. Bell et al., The journal of allergy and clinical immunology 2013;1:110-21; quiz 22; and Desai et al., Clinical and experimental immunology 2009;158:10-9. The collective data herein suggests that targeting the VNN1 pathway may be useful therapeutic strategy to enhance steroid response among these difficult to treat patients.

The data herein reveal that VNN1 is likely not a key contributor to inflammation in asthma since VNN1^{-/-} mice develop airway inflammation and AHR similar to wild type mice. Consistent with this, the expression level of VNN1 in mouse lungs was not altered by repeated allergen or IL-13 in experimental models of asthma. Zimmermann et al., Journal of immunology 2004;172:1815-24; and Lewis et al., The Journal of allergy and clinical immunology 2009;123:795-804 e8. Further, in patients, VNN1 expression levels were similar between children with asthma exacerbation, children with stable asthma, and non-asthmatic controls, supporting that VNN1 expression is not dysregulated in asthma.

Although not apparently necessary in the pathogenesis of asthma, VNN1 seems to play an important role in the host response to steroid treatment. VNN1 expression consistently increased following steroid treatment only in children who were good responders. Absence of the VNN1 gene resulted in resistance to steroid treatment that was reflected by persistent AHR and inflammatory cells in the lungs despite treatment with dexamethasone in mice. Notably, eosinophils---a hallmark of pediatric severe therapyresistant asthma (STRA), persisted in the BALF and lungs of the VNN1^{-/-} mice after dexamethasone treatment. Bossley et al., The Journal of allergy and clinical immunology, 2012;129:974-82 e13. Together, these findings suggest that VNN1 contributes to optimal host response to steroid treatment.

Without being bound by theory, the study herein suggests a model whereby steroid treatment induces methylation of VNN1 at CpG4 and this leads to increased expression of VNN1 in good responders, but not poor responders (Fig. 4). Methylation of CpG sites at promoter regions is generally believed to cause gene silencing (Baylin SB. Nature clinical practice Oncology 2005;2 Suppl 1: S4-11) either by prohibiting the binding of active transcription factor (TF) (Nan et al., Novartis Foundation symposium 1998;214:6-16; discussion -21, 46-50) or recruitment of chromatin modifiers to establish a repressive chromatin structure (Hon et al., Genome research 2012;22:246-58). However, positive correlations between promoter methylation and increased gene expression have been reported. Kulis et al., Nature genetics 2012;44:1236-42; and Wagner et al., Genome biology 2014;15: R37. A recent study has verified numerous transcription factors with methylated CpG (mCpG)-dependent DNA binding activity in human embryonic stem cells. Hu et al., eLife 2013;2:e00726. Further, a zinc finger protein, CTCF, was found to bind to the promoter region of VNN1 gene in small airway epithelial cells. Wang et al., Genome research 2012;22:1680-8. CTCF can function as a chromatin insulator, repressing gene expression by blocking the interaction between gene promoters and enhancer. Bell et al., Cell 1999;98:387-96. For example, CTCF binds to the imprinting control region (ICR) of the Igf2/H19 locus and silences Igf2 expression via its enhancer-blocking activity. This activity is sensitive to DNA methylation as methylation of CpGs within the ICR abolishes the binding of CTCF and thus allows Igf2 expression. Hark et al., Nature 2000;405:486-9; and Bell et al., Nature 2000;405:482-5. These findings provide possible explanations for the observation reported herein that the methylation level of the CpG4 motif was positively correlated with gene expression. It is conceivable that the methylated CpG4 site recruits mCpG-dependent transcription factors to the promoter, or abolishes the binding of an insulator such as CTCF to the promoter, allowing the mRNA expression of VNN1.

Anecdotally, there were 2 patients who were readmitted during the course of the study and these 2 patients maintained their responder phenotype, which was predicted by VNN1 T₀/T₁ expression. A retrospective analysis of medical records was also conducted for asthma hospital admissions during a period of three years. Here were 165 children aged 5-18 who had 2 or more admissions for asthma during this time frame (readmissions within 30 days were excluded) and who were poor treatment responders (according to the criteria used herein) on their first admission. Of these children, 129 (78.2%) remained poor responders at the subsequent admission, supporting that the poor responder phenotype is conserved over time.

In summary, VNN1 contributes to steroid response in asthma patients, such as children hospitalized for asthma. VNN1 expression and methylation at CpG4 at its promoter are shown to be clinically useful biomarkers of steroid responsiveness in asthma patients, including children hospitalized for asthma exacerbation.

The following items are herein described.
1. A method of assessing responsiveness to a steroid treatment of asthma in a subject, the method comprising:
   (a) measuring a level of Vanin 1 (VNN1) expression in a biological sample obtained from a subject having asthma, suspected of having asthma, or at risk for asthma; and
   (b) assessing the subject's responsiveness to the steroid treatment based on the level of VNN1 expression,
   wherein a decreased level of VNN1 expression relative to a pre-determined value indicates that the subject is not responsive or not likely to respond to the steroid treatment, and wherein a same or elevated level of VNN1 expression relative to the pre-determined value indicates that the subject is responsive or likely to respond to the steroid treatment.
2. The method of item 1, wherein the level of VNN1 expression is represented by a level of VNN1 mRNA in the biological sample of the subject.
3. The method of item 2, wherein the level of VNN1 mRNA is measured by an array-based assay or a PCR-based assay.
4. The method of item 1, wherein the level of VNN1 expression is represented by a level of CpG methylation in the promoter of a VNN1 gene.
5. The method of item 4, wherein the level of CpG methylation is the methylation level of CpG4 in the promoter of the VNN1 gene.
6. The method of item 4 or item 5, wherein the level of CpG methylation is measured by a bisulphite sequencing assay.
7. The method of item 6, wherein the bisulphite sequencing assay involves pyrosequencing.
8. The method of any of items 1-7, wherein the biological sample contains nasal epithelial cells.
9. The method of any of items 1 to 8, wherein the subject is a human asthma patient who has undergone or is undergoing a steroid treatment.
10. The method of item 9, further comprising:
   maintaining or repeating the steroid treatment, if the subject is responsive or likely to respond to the steroid treatment, or
   applying an alternative treatment to the subject, if the subject is not responsive or not likely to respond to the steroid treatment.
11. The method of item 10, wherein the alternative treatment is a non-steroid treatment.
12. The method of item 10, wherein the alternative treatment is a combined therapy comprising a non-steroid treatment and a steroid treatment.
13. The method of item 11 or item 12, wherein the non-steroid treatment involves a mast cell stabilizer, a leukotriene modifier, an immunomodulator, or a combination thereof.
14. The method of any of items 1-8, wherein the subject is a human patient free of steroid treatment.
15. The method of item 14, further comprising:
   applying a steroid treatment to the subject, if the subject is responsive or likely to respond to the steroid treatment; or
   applying a non-steroid treatment or a combined therapy comprising a non-steroid treatment to the subject, if the subject is not responsive or not likely to respond to the steroid treatment.
16. The method of item 15, wherein the non-steroid treatment involves a mast cell stabilizer, a leukotriene modifier, an immunomodulator, or a combination thereof.
17. The method of any of items 1-16, wherein the steroid treatment comprises prednisone, corticosteroid, methylprednisolone, dexamethasone, or a combination thereof.
18. The method of any of items 1-17, wherein the method further comprises monitoring development of an asthma symptom of the subject who is at risk for asthma, if the subject is not responsive or not likely to respond to a steroid treatment.
19. The method of any of items 1-18, wherein the method further comprises performing a home intervention to reduce the risk for asthma development, if the subject is not responsive or not likely to respond to a steroid treatment.
20. The method of any of items 1-19, wherein the method further comprises reducing environmental risk factors for asthma development, if the subject is not responsive or not likely to respond to a steroid treatment.
21. A method of treating a subject with asthma, the method comprising:
   (a) applying a steroid treatment to a subject in need thereof, wherein the subject exhibits the same or elevated level of VNN1 expression in nasal epithelial cells; or
   (b) applying a non-steroid treatment or a combined therapy comprising a non-steroid treatment and a steroid treatment to a subject in need thereof, wherein the subject exhibits a decreased level of VNN1 in nasal epithelial cells.
22. The method of item 21, wherein the level of VNN1 expression is represented by the level of VNN1 mRNA or the level of CpG methylation in the promoter of a VNN1 gene.
23. The method of item 22, wherein the level of VNN1 expression is represented by the level of methylation at the CpG4 site in the promoter of the VNN1 gene.
24. The method of any of items 21-23, wherein the subject is a human patient suffering from an asthma exacerbation.
25. The method of any of items 21-24, wherein the subject is a child who is 18 years old or younger.
26. A kit for determining a level of VNN1 expression in a biological sample, the kit comprising one or more agents for measuring the level of VNN1 expression, wherein the one or more agents are:
   (i) an antibody specifically binding to VNN1 protein;
   (ii) one or more oligonucleotides, at least one being complementary to a region within the mRNA of VNN1; or
   (iii) bisulfite and one or more oligonucleotides for use in bisulfite sequencing.
27. A method of treating a subject with asthma, the method comprising:
   administering an effective amount of cysteamine or a pharmaceutically acceptable salt thereof to a subject having asthma, wherein the subject does not respond to or is not likely to respond to a steroid treatment.
28. The method of item 27, wherein the subject has a decreased level of VNN1 expression relative to a pre-determined value.
29. The method of item 28, wherein the decreased level of VNN1 expression is represented by a decreased level of VNN1 mRNA or a decreased level of VNN1 protein in a biological sample of the subject.
30. The method of item 28, wherein the decreased level of VNN1 expression is represented by a decreased level of CpG methylation in the promoter of a VNN1 gene.
31. The method of item 30, wherein the level of CpG methylation is the methylation level of CpG4 in the promoter of the VNN1 gene.
32. The method of any of items 27-31, wherein the pharmaceutically acceptable salt of cysteamine is cysteamine bitartrate or cysteamine hydrochloride.
33. The method of any of items 27-31, wherein the cysteamine is in disulfide form.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present disclosure, and without departing from the spirit and scope thereof, can make various changes and modifications of the disclosure to adapt it to various usages and conditions. Thus, other embodiments are also within the claims.

### EQUIVALENTS

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

All definitions, as defined and used herein, should be understood to control over dictionary definitions, definitions in documents incorporated by reference, and/or ordinary meanings of the defined terms.

All references, patents and patent applications disclosed herein are incorporated by reference with respect to the subject matter for which each is cited, which in some cases may encompass the entirety of the document.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

## Claims

1. Cysteamine or a pharmaceutically acceptable salt for use in treating asthma in a subject, wherein the subject does not respond to or is not likely to respond to a steroid treatment.

2. The cysteamine or the pharmaceutically acceptable salt for use of claim 1, wherein the pharmaceutically acceptable salt is cysteamine bitartrate or cysteamine hydrochloride.

3. The cysteamine or the pharmaceutically acceptable salt for use of claim 1, wherein the cysteamine is in disulfide form.

4. The cysteamine or the pharmaceutically acceptable salt for use of claim 1, wherein the subject is a human asthma patient.

5. The cysteamine or the pharmaceutically acceptable salt for use of claim 4, wherein the human asthma patient is a child patient.
